# NEW EUROPEAN PATENT SPECIFICATION

(11) **EP 3 269 717 B2**
(45) Date of publication and mention of the opposition decision: **14.06.2023**
(45) Mention of the grant of the patent: 27.02.2019
(21) Application number: 17182561.5
(22) Date of filing: 08.10.2010
(51) Int. Cl.: C07D 471/08, A61K 31/439, A61K 31/551, A61P 31/04

(54) **POLYMORPHIC AND PSEUDOPOLYMORPHIC FORMS OF PHARMACEUTICAL COMPOUND NXL104**
POLYMORPHE UND PSEUDOPOLYMORPHE FORMEN DER PHARMAZEUTISCHEN VERBINDUNG NXL104
FORMES POLYMORPHES ET PSEUDOPOLYMORPHES DU COMPOSÉ PHARMACEUTIQUE NXL104

(30) Priority: 09.10.2009 FR 0904864; 23.11.2009 US 263663 P
(43) Date of publication of application: 17.01.2018
(62) Divisional of application: 10762934.7
(73) Proprietor: PFIZER IRELAND PHARMACEUTICALS, Ringaskiddy Cork (IE)
(72) Inventor: BHATTACHARYA, Sisir, Hauppauge, NY New York 11788 (US); BONNET, Alain, 02400 Chateau-Thierry (FR); DEDHIYA, Mahendra G., Pomona, NY New York 10970 (US); DUCANDAS, Véronique, 94400 Vitry Sur Seine (FR); GIULIANI, Alexandre, 94440 Villecresnes (FR); PRIOUR, Alain, 75019 Paris (FR); RAVAUX, Valérie, 01600 Reyrieux (FR); SPARGO, Peter, Deal, Kent CT14 9QT (GB)
(74) Representative: Pfizer

(56) References cited:
- EP-A1- 2 135 959
- WO-A1-02/10172
- BONNEFOY ALAIN ET AL: "In vitro activity of AVE1330A, an innovative broad-spectrum non-.beta.-lactam .beta.-lactamase inhibitor", JOURNAL OF ANTIMICROBIAL CHEMOTHERAPY, vol. 54, no. 2, 2004, pages 410-417, XP002582639,
- LIVERMORE DAVID M. ET AL: "NXL104 combinations versus Enterobacteriaceae with CTX-M extended-spectrum .beta.-lactamases and carbapenemases", JOURNAL OF ANTIMICROBIAL CHEMOTHERAPY, vol. 62, no. 5, 2008, pages 1053-1056, XP002582640,

## Description

The present invention relates to a polymorphic form of the sodium salt of (1R,2S,5R)-7-oxo-6-sulfooxy-1,6-diazabicyclo[3.2.1]octane-2-carboxamide in crystallized form and processes for the preparation of said crystalline form, pharmaceutical compositions comprising the crystalline form alone or in combination with an antibacterial agent (e.g. ceftazidime, ceftaroline fosamil), and said crystalline form in combination with an antibacterial agent (e.g. ceftazidime, ceftaroline fosamil) for use in the treatment of bacterial infections.

Application WO 02/10172 describes the production of azabicyclic compounds and salts thereof with acids and bases, and in particular trans-7-oxo-6-sulfooxy-1,6-diazabicyclo[3.2.1]octane-2-carboxamide and its pyridinium, tetrabutylammonium and sodium salts. Application WO 03/063864 describes the use of compounds including trans-7-oxo-6-sulfooxy-1,6-diazabicyclo[3.2.1]octane-2-carboxamide sodium salt, as β-lactamase inhibitors, and the use of said β-lactamase inhibitors in combination with β-lactamine antibiotics such as ceftazidime.

In WO 02/10172 the preparation of the racemic sodium salt of trans-7-oxo-6-sulfooxy-1,6-diazabicyclo[3.2.1]octane-2-carboxamide is described, it being obtained indirectly from a compound described in Example 33b of WO 02/10172, by exchange of the tetrabutylammonium counter-ion with sodium, eluting an aqueous solution of the salt on ion exchange resin, treated beforehand with sodium hydroxide.

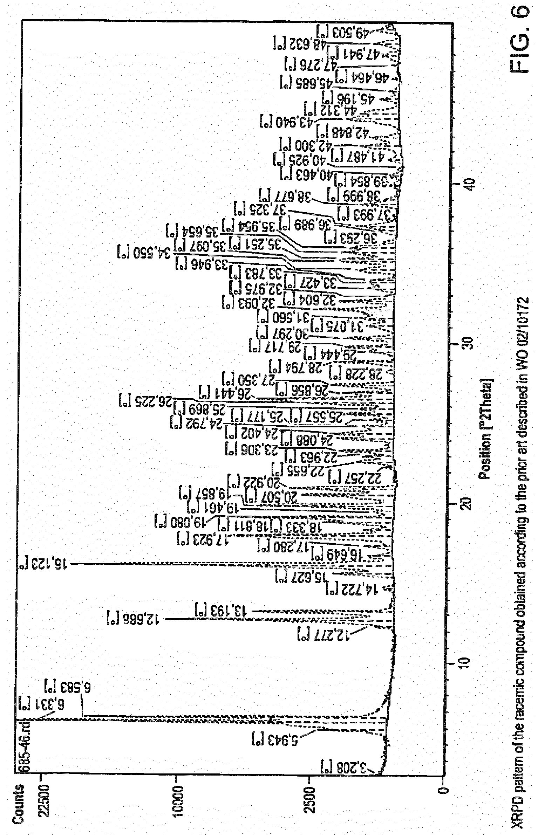

The sodium salt is obtained in solid form, after elimination of the water. The racemic product crystallizes as mentioned in Example 33c of WO 02/10172 and has been characterized by X-ray powder diffraction (XRPD) analysis of a sample prepared hereafter in the experimental section (see Example 7 and Figure 6).

It was found that only one enantiomer was active and therefore there was a need to use just the active enantiomer which is the sodium salt of (1R,2S,5R)-7-oxo-6-sulfooxy-1,6-diazabicyclo[3.2.1]octane-2-carboxamide, (also known as NXL104). Concentration to dryness is carried out in the laboratory by evaporation. In practice, the water is removed by lyophilization to obtain a homogeneous solid form. However it was found that the amorphous form of NXL104 was not very stable in the presence of water and is hygroscopic and of low density, which makes it difficult to handle and store, and consequently makes it difficult to scale up its method of preparation to an industrial level. In itself, lyophilization carried out in the laboratory is already a technique that is difficult to scale up to the industrial level. Moreover, the method of ion exchange on resin as described for the preparation of the starting material for the racemate and as described in Example 10 is expensive and of low productivity on account of the large amounts of resin, the dilution with water that is necessary for quantitative ion exchange, the very long duration of the operation and the high energy costs required, and for these reasons as well, the method would be difficult to use on an industrial scale.

The sodium salt of (1R,2S,5R)-7-oxo-6-sulfooxy-1,6-diazabicyclo[3.2.1]octane-2-carboxamide is a beta-lactamase inhibitor, which reacts with a protein, forming a covalent bond. This reactive inhibitor, a consequence of the internal strain of the N-oxosulfoxyurea ring, is intrinsically sensitive to moisture and to heat, just like the β-lactams, although it is not a β-lactam. The main manner of degradation of the sodium salt of (1R,2S,5R)-7-oxo-6-sulfooxy-1,6-diazabicyclo[3.2.1]octane-2-carboxamide is by hydrolysis of the N-oxosulfoxyurea ring. To minimize degradation, it is advantageous to isolate this molecule at room temperature or at low temperature and minimize the duration of exposure in aqueous solution. These conditions are fulfilled during crystallization or lyophilization but are difficult to fulfil during concentration of an aqueous solution to dryness, as described in Application WO 02/10172. In practice, the aqueous solution containing the sodium salt of (1R,2S,5R)-7-oxo-6-sulfooxy-1,6-diazabicyclo[3.2.1]octane-2-carboxamide can only be concentrated by lyophilization, in order to obtain the product cleanly in the amorphous form.

The present invention relates to the sodium salt of (1R,2S,5R)-7-oxo-6-sulfooxy-1,6-diazabicyclo[3.2.1]octane-2-carboxamide of formula (I) 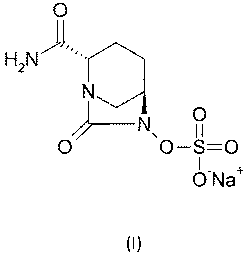 in crystallised form, anhydrous, characterized in that the salt is in its polymorphic form "B", having an X-ray diffraction pattern with at least five characteristic peaks at 13.0 +/- 0.5 degrees 2θ, at 16.5 +/- 0.5 degrees 2θ, at 17.2 +/- 0.5 degrees 2θ, at 17.5 +/- 0.5 degrees 2θ, at 22.3 +/- 0.5 degrees 2θ and two specific peaks at 10.4 +/- 0.5 degrees 2θ and at 13.0 +/-0.5 degrees 2θ. either anhydrous as are "B" and "D" or are hydrates as are "A" and "E".

A fifth form of NXL104, "Form C" is also described herein but has been observed only as a mixture with form A.

The present invention further relates to novel and improved methods of preparation of form B of the sodium salt of the (1R,2S,5R)-7-oxo-6-sulfooxy-1,6-diazabicyclo[3.2.1]octane-2-carboxamide enantiomer making it possible to obtain said salt in perfectly crystallized and stable form, without having recourse to the ion exchange technique nor to lyophilization under the non-industrial conditions described above. The methods according to the invention therefore offer the dual advantages of simplifying the techniques and permitting their scale up to the industrial level, while supplying in a reproducible way a crystallized form that is stable, easy to isolate, handle, store and formulate.

A crystallized monohydrated sodium salt of formula (I), "Form A", can be defined as having:
an X-ray powder diffraction pattern with at least one characteristic peak at about 8.5 +/- 0.5 degrees 2θ; or
an X-ray powder diffraction pattern with at least one characteristic peak at about 15.3 +/- 0.5 degrees 2θ; or
an X-ray powder diffraction pattern with at least one characteristic peak at about 16.4 +/- 0.5 degrees 2θ; or
an X-ray powder diffraction pattern with at least one characteristic peak at about 17.0 +/- 0.5 degrees 2θ; or
an X-ray powder diffraction pattern with at least one characteristic peak at about 24.3 +/- 0.5 degrees 2θ; or
an X-ray powder diffraction pattern with at least two characteristic peaks at about 8.5 +/- 0.5 degrees 2θ and at about 15.3 +/- 0.5 degrees 2θ; or
an X-ray powder diffraction pattern with at least three characteristic peaks at about 8.5 +/- 0.5 degrees 2θ, at about 15.3 +/- 0.5 degrees 2θ and at about 16.4 +/- 0.5 degrees 2θ; or
an X-ray powder diffraction pattern with at least four characteristic peaks at about 8.5 +/- 0.5 degrees 2θ, at about 15.3 +/- 0.5 degrees 2θ, at about 16.4 +/- 0.5 degrees 2θ and at about 17.0 +/- 0.5 degrees 2θ; or
an X-ray powder diffraction pattern with at least five characteristic peaks at about 8.5 +/- 0.5 degrees 2θ, at about 15.3 +/- 0.5 degrees 2θ, at about 16.4 +/- 0.5 degrees 2θ, at about 17.0 +/- 0.5 degrees 2θ and at about 24.3 +/- 0.5 degrees 2θ.
an X-ray powder diffraction pattern with five characteristic lines at 2θ (± 0.5°) 8.48, 15.34, 16.38, 17.04, 24.28 and a specific line at 8.48; or
an X-ray powder diffraction pattern with five characteristic lines at 2θ (± 0.1°) 8.48, 15.34, 16.38, 17.04, 24.28 and a specific line at 8.48; or
an X-ray powder diffraction pattern comprising a characteristic peak at about 8.5; about 15.3; about 16.4; about 17.0; or about 24.3 or a combination thereof wherein each value may be +/-0.5 degrees 2θ.

A crystallized dihydrated sodium salt of formula (I), "Form E", can be defined as having:
an X-ray powder diffraction pattern with at least one characteristic peak at about 13.7 +/- 0.5 degrees 2θ; or
an X-ray powder diffraction pattern with at least one characteristic peak at about 15.0 +/- 0.5 degrees 2θ; or
an X-ray powder diffraction pattern with at least one characteristic peak at about 15.4 +/- 0.5 degrees 2θ; or
an X-ray powder diffraction pattern with at least one characteristic peak at about 15.7 +/- 0.5 degrees 2θ; or
an X-ray powder diffraction pattern with at least one characteristic peak at about 19.4 +/- 0.5 degrees 2θ; or
an X-ray powder diffraction pattern with at least one specific peak at about 24.6 +/-0.5 degrees 2θ; or
an X-ray powder diffraction pattern with at least two specific peaks at about 15.0 +/-0.5 degrees 2θ and 24.6 +/- 0.5 degrees 2θ; or
an X-ray powder diffraction pattern with at least two characteristic peaks at about 13.7 +/- 0.5 degrees 2θ and at about 15.0 +/- 0.5 degrees 2θ; or
an X-ray powder diffraction pattern with at least three characteristic peaks at about 13.7 +/- 0.5 degrees 2θ, at about 15.0 +/- 0.5 degrees 2θ and at about 15.4 +/- 0.5 degrees 2θ; or
an X-ray powder diffraction pattern with at least four characteristic peaks at about 13.7 +/- 0.5 degrees 2θ, at about 15.0 +/- 0.5 degrees 2θ, at about 15.4 +/- 0.5 degrees 2θ and at about 15.7 +/- 0.5 degrees 2θ; or
an X-ray powder diffraction pattern with at least five characteristic peaks at about 13.7 +/- 0.5 degrees 2θ, at about 15.0 +/- 0.5 degrees 2θ, at about 15.4 +/- 0.5 degrees 2θ, at about 15.7 +/- 0.5 degrees 2θ and at about 19.4 +/- 0.5 degrees 2θ; or
an X-ray powder diffraction pattern with five characteristic lines at 2θ (± 0.5°) 13.65, 15.01, 15.38, 15.72, 19.42 and two specific lines at 15.01 and 24.57; or
an X-ray diffraction pattern with five characteristic lines at 2θ (± 0.1°) 13.65, 15.01, 15.38, 15.72, 19.42 and two specific lines at 15.01 and 24.57; or
an X-ray powder diffraction pattern comprising a characteristic peak at about 13.7; about 15.0; about 15.4; about 15.7; or about 19.4; a combination thereof wherein each value may be +/-0.5 degrees 2θ.

A crystallized anhydrous sodium salt of formula (I), "Form B", can be defined as having:
an X-ray powder diffraction pattern with at least one characteristic peak at about 13.0 +/- 0.5 degrees 2θ; or
an X-ray powder diffraction pattern with at least one characteristic peak at about 16.5 +/- 0.5 degrees 2θ; or
an X-ray powder diffraction pattern with at least one characteristic peak at about 17.2 +/- 0.5 degrees 2θ; or
an X-ray powder diffraction pattern with at least one characteristic peak at about 17.5 +/- 0.5 degrees 2θ; or
an X-ray powder diffraction pattern with at least one characteristic peak at about 22.3 +/- 0.5 degrees 2θ; or
an X-ray powder diffraction pattern with at least one specific peak at about 10.4 +/-0.5 degrees 2θ; or
an X-ray powder diffraction pattern with at least two specific peaks at about 10.4 +/-0.5 degrees 2θ and at about 13.0 +/- 0.5 degrees 2θ; or
an X-ray powder diffraction pattern with at least two characteristic peaks at about 13.0 +/- 0.5 degrees 2θ and at about 16.5 +/- 0.5 degrees 2θ; or
an X-ray powder diffraction pattern with at least three characteristic peaks at about 13.0 +/- 0.5 degrees 2θ, at about 16.5 +/- 0.5 degrees 2θ and at about 17.2 +/- 0.5 degrees 2θ; or
an X-ray powder diffraction pattern with at least four characteristic peaks at about 13.0 +/- 0.5 degrees 2θ, at about 16.5 +/- 0.5 degrees 2θ, at about 17.2 +/- 0.5 degrees 2θ and at about 17.5 +/- 0.5 degrees 2θ; or
an X-ray powder diffraction pattern with at least five characteristic peaks at about 13.0 +/- 0.5 degrees 2θ, at about 16.5 +/- 0.5 degrees 2θ, at about 17.2 +/- 0.5 degrees 2θ, at about 17.5 +/- 0.5 degrees 2θ and at about 22.3 +/- 0.5 degrees 2θ; or
an X-ray powder diffraction pattern with at least five characteristic peaks at about 13.0 +/- 0.5 degrees 2θ, at about 16.5 +/- 0.5 degrees 2θ, at about 17.2 +/- 0.5 degrees 2θ, at about 17.5 +/- 0.5 degrees 2θ, at about 22.3 +/- 0.5 degrees 2θ and two specific peaks at about 10.4 +/- 0.5 degrees 2θ and at about 13.0 +/-0.5 degrees 2θ; or
an X-ray powder diffraction pattern with five characteristic lines at 2θ (± 0.5°) 12.97, 16.45, 17.24, 17.45, 22.29 and two specific lines at 10.36 and 12.97; or
an X-ray powder diffraction pattern with five characteristic lines at 2θ (± 0.1°) 12.97, 16.45, 17.24, 17.45, 22.29 and two specific lines at 10.36 and 12.97; or
an X-ray powder diffraction pattern comprising a characteristic peak at about 13.0; about 16.5; about 17.2; about 17.5; or about 22.3 or a combination thereof wherein each value may be +/-0.5 degrees 2θ.

A crystallized anhydrous sodium salt of formula (I), "Form D", can be defined as having:
an X-ray powder diffraction pattern with at least one characteristic peak at about 16.2 +/- 0.5 degrees 2θ; or
an X-ray powder diffraction pattern with at least one characteristic peak at about 17.4 +/- 0.5 degrees 2θ; or
an X-ray powder diffraction pattern with at least one characteristic peak at about 17.8 +/- 0.5 degrees 2θ; or
an X-ray powder diffraction pattern with at least one characteristic peak at about 18.5 +/- 0.5 degrees 2θ; or
an X-ray powder diffraction pattern with at least one characteristic peak at about 22.2 +/- 0.5 degrees 2θ; or
an X-ray powder diffraction pattern with at least one specific peak at about 12.4 +/-0.5 degrees 2θ; or
an X-ray powder diffraction pattern with at least two characteristic peaks at about 16.2 +/- 0.5 degrees 2θ and at about 17.4 +/- 0.5 degrees 2θ; or
an X-ray powder diffraction pattern with at least three characteristic peaks at about 16.2 +/- 0.5 degrees 2θ, at about 17.4 +/- 0.5 degrees 2θ and at about 17.8 +/- 0.5 degrees 2θ; or
an X-ray powder diffraction pattern with at least four characteristic peaks at about 16.2 +/- 0.5 degrees 2θ, at about 17.4 +/- 0.5 degrees 2θ, at about 17.8 +/- 0.5 degrees 2θ and at about 18.5+/- 0.5 degrees 2θ; or
an X-ray powder diffraction pattern with at least five characteristic peaks at about 16.2 +/- 0.5 degrees 2θ, at about 17.4 +/- 0.5 degrees 2θ, at about 17.8 +/- 0.5 degrees 2θ, at about 18.5 +/- 0.5 degrees 2θ and at about 22.2 +/- 0.5 degrees 2θ; or
an X-ray powder diffraction pattern with at least five characteristic peaks at about 16.2 +/- 0.5 degrees 2θ, at about 17.4 +/- 0.5 degrees 2θ, at about 17.8 +/- 0.5 degrees 2θ, at about 18.5 +/- 0.5 degrees 2θ, at about 22.2 +/- 0.5 degrees 2θ and one specific peak at about 12.4 +/- 0.5 degrees 2θ; or
an X-ray powder diffraction pattern with five characteristic lines at 2 0 (± 0.5°) 16.23, 17.44, 17.75, 18.53, 22.22 and a specific line at 12.43; or
an X-ray powder diffraction pattern with five characteristic lines at 2θ (± 0.1°) 16.23, 17.44, 17.75, 18.53, 22.22 and a specific line at 12.43; or
an X-ray powder diffraction pattern comprising a characteristic peak at about 12.4; about 16.2; about 17.4; about 17.8; about 18.5; or about 22.2; or a combination thereof wherein each value may be +/-0.5 degrees 2θ.

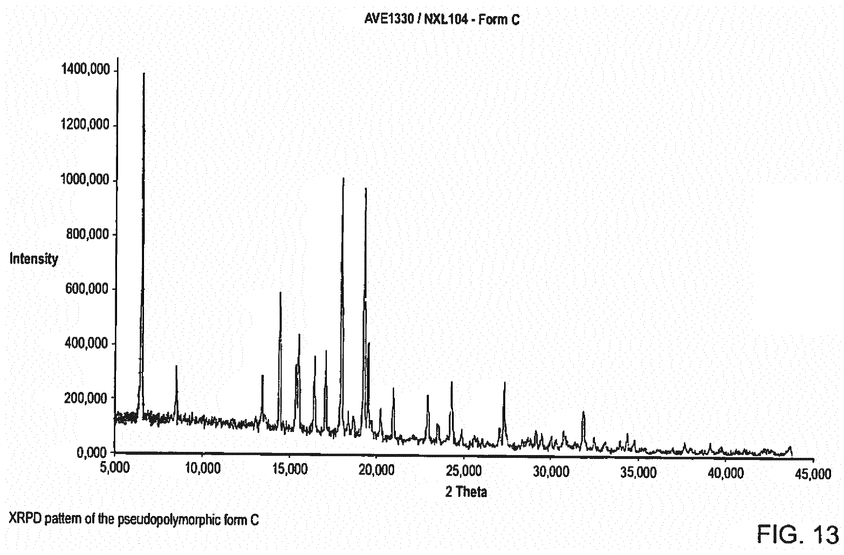

A crystallized sodium salt of formula (I), "Form C", is not isolated as a pure form but is obtained in a mixture with one or more other forms, in particular Form A. An X-ray powder diffraction pattern was obtained for the mixture of forms including Form C and is shown in Figure 13. It has characteristic peaks at about 6.5; about 8.5; about 13.4; about 14.4; about 15.4; about 15.5; about 16.4; about 17.1; about 18.0; about 19.3; about 19.5; about 21.0; about 22.9; about 24.3; about 27.3 or about 31.9 +/-0.5 degrees 2θ or a combination thereof.

The Form C mixture can be characterized by:
an X-Ray powder diffraction pattern comprising a characteristic peak at about 6.5 +/-0.5 degrees 2θ;
an X-Ray powder diffraction pattern comprising a characteristic peak at about 18.0 +/- 0.5 degrees 2θ; or
an X-Ray powder diffraction pattern comprising a characteristic peak at about 19.3 +/- 0.5 degrees 2θ; or
an X-Ray powder diffraction pattern comprising a characteristic peak at about 14.4; about 15.5; about 16.4; about 17.1 or about 19.5 +/- 0.5 degrees 2θ or a combination thereof; or
an X-Ray powder diffraction pattern comprising a characteristic peak at about 8.5; about 13.4; about 15.4; about 21.0; about 22.9; about 24.3; about 27.3 or about 31.9 +/- 0.5 degrees 2θ or a combination thereof; or
an X-Ray powder diffraction pattern comprising characteristic peaks at about 6.5; about 8.5; about 13.4; about 14.4; about 15.4; about 15.5; about 16.4; about 17.1; about 18.0; about 19.3; about 19.5; about 21.0; about 22.9; about 24.3; about 27.3 and about 31.9 +/- 0.5 degrees 2θ.

A method for the preparation of the sodium salt of the (1R,2S,5R)-7-oxo-6-sulfooxy-1,6-diazabicyclo[3.2.1]octane-2-carboxamide enantiomer of formula (I): 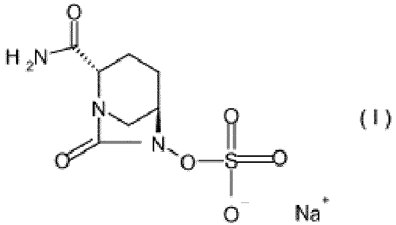 as defined above, is characterized in that the tetrabutylammonium salt of (1R,2S,5R)-7-oxo-6-sulfooxy-1,6-diazabicyclo[3.2.1]octane-2-carboxamide is treated in a (1-6 C) alkanol containing between 0 and 50% water, with a sodium salt that is soluble in the reaction mixture, and then the crystals obtained are isolated.

The sodium salt used is in particular an acetate, a butyrate, a hexanoate, an ethyl-hexanoate or a dodecylsulfate, and very preferably 2-ethyl-hexanoate.

The process of the reaction is an equilibrium that is displaced by the crystallization of the sodium salt, which can be applied advantageously on an industrial scale, making the method particularly useful.

Either the alcoholic solution of sodium 2-ethylhexanoate is added to the alcoholic solution of the tetrabutylammonium salt, or vice versa.

The (1-6 C) alkanol used in the method according to the invention is preferably ethanol, propanol or linear or branched butanol, and very preferably ethanol. The operation is carried out in the presence of 0 to 10% water, at a temperature between 15 and 40°C.

The invention in particular relates to a method as defined above, for the preparation of the sodium salt of formula (I), in the anhydrous polymorphic form, "Form B", as described herein, characterized in that a solution of sodium 2-ethylhexanoate in pure ethanol is added to a solution of the tetrabutylammonium salt of (1R,2S,5R)-7-oxo-6-sulfooxy-1,6-diazabicyclo[3.2.1]octane-2-carboxamide in an ethanol/water mixture in such a way that the final proportion of water is from 0 to 5 wt.% of the solvent, operating at a temperature from 10 to 40°C, in the presence of seed crystals of the polymorphic "Form B" or of the pseudopolymorphic "Form A" as described herein.

The parameters, such as the proportion of water in the reaction mixture, the duration of addition, the temperature and the concentration are all relevant in determining the crystalline form that is obtained. In order to obtain the pure B form, it is preferable to operate in the presence of seed crystals of the polymorphic "Form B" and of a final proportion of water less than 2%, introducing the solution of sodium 2-ethylhexanoate over a period of 1 to 7 hours and operating at a temperature from 10 to 40°C, and very preferably 30 to 35°C.

The invention also in particular relates to a method as defined above, for the preparation of the sodium salt of formula (I), in the anhydrous polymorphic "Form B", characterized in that an ethanolic solution of the tetrabutylammonium salt of (1R,2S,5R)-7-oxo-6-sulfooxy-1,6-diazabicyclo[3.2.1]octane-2-carboxamide is added to an ethanol/water mixture of sodium 2-ethylhexanoate, moreover operating under the same conditions of solvent and temperatures as those described above.

A method as defined above, for the preparation of the sodium salt of formula (I), in its monohydrated pseudopolymorphic form "Form A" as described herein, is characterized in that a solution of sodium 2-ethylhexanoate in pure ethanol is added to a solution of the tetrabutylammonium salt of (1R,2S,5R)-7-oxo-6-sulfooxy-1,6-diazabicyclo[3.2.1]octane-2-carboxamide in an ethanol/water mixture in such a way that the final proportion of water is from 3 to 10 wt.% of the solvent, operating at a temperature from 10 to 40°C. Crystallization is carried out in the absence of seed crystals or by adding seed crystals of the pseudopolymorphic "Form A".

The parameters, such as the proportion of water in the reaction mixture, the duration of addition, the temperature and the concentration act interdependently on the crystalline form. In order to obtain the pure A form, it is preferable to operate at a temperature from 20 to 35°C and very preferably at room temperature, in the presence of seed crystals of the pseudopolymorphic "Form A", a final proportion of water greater than 5 wt.% of the solvent, and introducing the solution of sodium 2-ethylhexanoate over a period of 30 minutes to 2 hours.

Another method as defined above, for the preparation of the sodium salt of formula (I), in the monohydrated pseudopolymorphic "Form A", is characterized in that an ethanolic solution of the tetrabutylammonium salt of (1R,2S,5R)-7-oxo-6-sulfooxy-1,6-diazabicyclo[3.2.1]octane-2-carboxamide is added to an ethanol/water mixture of sodium 2-ethylhexanoate, operating under the same conditions of solvent and temperatures as those described above.

A method as defined above, for the preparation of the sodium salt of formula (I), in its anhydrous polymorphic form "Form D" as described herein, is characterized in that an ethanolic solution of sodium 2-ethylhexanoate is added to an ethanolic solution of the tetrabutylammonium salt of (1R,2S,5R)-7-oxo-6-sulfooxy-1,6-diazabicyclo[3.2.1]octane-2-carboxamide, operating at room temperature. Crystallization is carried out in the absence of seed crystals or by adding seed crystals of the polymorphic "Form D" or optionally of the pseudopolymorphic "Form A".

The parameters such as the proportion of water in the reaction mixture, the duration of addition, the temperature and the concentration act interdependently on the crystalline form. In order to obtain the pure D form, it is preferable to operate in the absence of seed crystals, introducing the solution of sodium 2-ethylhexanoate over a period of 30 minutes or less, and operating at room temperature.

Another method as defined above, for the preparation of the sodium salt of formula (I), in its polymorphic "Form D", is characterized in that an ethanolic solution of the tetrabutylammonium salt of (1R,2S,5R)-7-oxo-6-sulfooxy-1,6-diazabicyclo[3.2.1]octane-2-carboxamide is added to an ethanolic solution of sodium 2-ethylhexanoate, operating under the same conditions of solvent and temperature as those described above.

Another dihydrated pseudopolymorphic form called "Form E" as described herein was obtained by a method that is characterized in that crystals of "Form A" are suspended in water, and the suspension is then left to evaporate slowly in a humid atmosphere. Crystals have also been obtained by trituration of crystals of "Form A" in water or in an alkanol-water mixture, or by conversion, in a humid atmosphere, of the anhydrous "Form B" and "Form D" to the monohydrated "Form A" and then to the dihydrated "Form E". This "Form E" is particularly stable at higher humidities above 70% Relative Humidity. Form C is anhydrous and highly hygroscopic since it converts to monohydrated "Form A" at Relative Humidity as low as 5%.

The polymorphic and pseudopolymorphic forms "A", "B", "D" and "E" have never been observed during the preparation of the sodium salt of the racemic compound trans-7-oxo-6-sulfooxy-1,6-diazabicyclo[3.2.1]octane-2-carboxamide described in Application WO 02/10172. Analysis of the monocrystal of this material, as prepared and described in Example 8 below, shows the presence of both enantiomers and two molecules of water within the unit cell, characteristic of a dihydrated racemic compound. The higher water solubility of the enantiomer compared with the racemate makes it highly unlikely that any of the enantiomeric forms could ever be obtained by concentration and crystallisation from water. Nor can they be prepared on an industrial scale under the conditions described in Application WO 02/10172, as the excessively dilute aqueous solutions resulting from ion exchange and the stability of the sodium salt of (1R,2S,5R)-7-oxo-6-sulfooxy-1,6-diazabicyclo[3.2.1]octane-2-carboxamide do not permit their concentration and their crystallization by evaporation of the water.

Seed crystals of the pseudopolymorphic "Form A" were obtained by adding, over forty-five minutes, 19 volumes of ethanol to a solution of the amorphous sodium salt of (1R,2S,5R)-7-oxo-6-sulfooxy-1,6-diazabicyclo[3.2.1]octane-2-carboxamide in one volume of water, cooling to 5°C in one hour and then holding at this temperature, filtration and finally drying.

Seed crystals of the polymorphic "Form B" were obtained by dissolving the amorphous salt in 33 volumes of methanol, adding 10 volumes of ethanol at 60°C, concentration of the solution to about 10 volumes at room temperature and then distillation of the methanol to constant volume, still at room temperature, with ethanol (25 volumes are added). The polymorphic "Form B" thus obtained was filtered and then dried.

The polymorphic and pseudopolymorphic forms "A", "B", "D" and "E" are stable and homogeneous, which is particularly important for their preparation on an industrial scale, as well as for their storage and their utilization in the formulation process.

It has been found that Form D crystals are very small making filtration difficult and slow and hence making it difficult to prepare Form D.

It has also been found that Form E is somewhat less stable because it tends to lose water and hydrolyse during long storage and at higher temperature.

If the relative humidity is controlled between 0-70% Relative Humidity and in the absence of gas flow Form A is a stable form.

In anhydrous conditions or low relative humidity below 60% Relative Humidity then Form B is a stable form.

Thus Form B is the most preferred form.

However Form B is not easy to prepare because in the absence of seed, if water is merely excluded, or under rapid crystallisation then kinetic Form D may be produced rather than Form B, for example see Example 5 hereinafter.

Surprisingly we have found that it is better to use some water in the method for preparing the anhydrous Form B. If too much water is used then Form A will be produced, such as in Examples 3 and 4. The range of water that produces Form B is relatively narrow.

In general, the obvious way to 'direct' crystallization to a particular form is to seed it with that form, however we have found that seeding with form A can generate forms B, D and E, see Examples 2,5, and 6. Thus very unusually in this case, seeding alone is not sufficient to achieve a particular crystalline form.

Surprisingly therefore of the crystalline forms "A", "B", "D" and "E" (which are all more stable than the amorphous form of NXL104), Form B is the most preferred form. Form B is anhydrous but again surprisingly the present invention describes a robust reproducible process for the preparation of Form B, that can be scaled up to an industrial level, and yet rather than being completely anhydrous said process uses some water or long addition time to minimise risk of obtaining non desirable form D.

The pseudopolymorphic "Form A" is a monohydrate (theoretical water content 5.90%) by weight) and the pseudopolymorphic "Form E" is a dihydrate. By coupling thermogravimetric analysis (TGA) with differential thermal analysis (SDTA) at 10°C/min, the pseudopolymorphic "Form A" displays a weight loss of 5.7%> at approximately 110°C, corresponding to the dehydration of the salt, followed by a decomposition exotherm with weight loss between 220 and 240°C. By the same technique, the pseudopolymorphic "Form E" displays a first weight loss of 5% at approximately 60°C and then a second weight loss of 5% at approximately 100°C before decomposition between 220 and 240°C. This loss of water in 2 stages corresponds to a dihydrated form with two non-equivalent molecules of water in the crystal lattice.

The polymorphic forms "B" and "D" are anhydrous, a maximum amount of water from 0 to 0.6% having been detected by Karl Fischer analysis in a product of "Form B" prepared as described later in the experimental section. The polymorphic forms "B" and "D" display an exothermic decomposition peak between 220 and 240°C measured by DSC (Differential Scanning Calorimetry).

The polymorphic and pseudopolymorphic forms "A", "B", "D" and "E" according to the invention are moreover characterized by the X-ray spectra ("XRPD diffraction pattern") as presented below and quite particularly by specific characteristic lines shown in the tables hereinafter.

The experimental powder diffraction patterns were obtained by diffraction of X-rays on powder in an X'pert Pro Philips instrument with the Kα radiation of copper (λ=1.5406A). The samples, without grinding, are put on a glass plate and are analyzed at ambient temperature and humidity with an angle 2θ from 5 to 50°. The characteristic peaks of each form were determined from the five lines that are generally the most intense. The specific peaks of each form were only detected in the polymorphic forms according to the invention. The mean value of each peak and its standard deviation were calculated from the experimental values of representative samples of each form.

The crystal structures of the monocrystals of the E and racemic dihydrate forms were obtained at 296K on a Rigaku Rapid R axis diffractometer equipped with a rotating copper anode (I=1.5406Å). The crystals structures of monocrystal of the A form were obtained at 233K on a Bruker Nonius diffractometer with the Kα radiation of molybdenum (I=0.7093A). Powder diffraction patterns are normally measured using copper Kα radiation. For comparison with the experimental powder patterns, the theoretical powder diffraction patterns for the pseudopolymorphic forms A and E and of the racemic dihydrate were calculated from the corresponding crystal structure data using the appropriate value for copper Kα radiation (1.5406Å).

In the attached drawings, Figures 1 to 5 show the experimental XRPD diffraction patterns of the polymorphic and pseudopolymorphic forms A, B, D and E, as well as the specific lines of these forms.

Figure 6 shows the XRPD diffraction pattern of the racemic compound described in Application WO 02/10172.

Figure 7 shows the theoretical XRPD diffraction pattern of the dihydrated form of the racemic compound (monocrystal prepared below in Example 8).

Figure 8 shows a comparison of the XRPD diffraction patterns of the racemic form versus the crystalline forms A, B, D, E.

Figures 9 and 10 show a comparison of the XRPD diffraction patterns of the racemic form versus the dihydrated form of the racemic compound (monocrystal prepared below in Example 8).

Figure 11 shows a representation of the crystal lattice of the monocrystal of the dihydrate racemate.

Figure 12 shows a comparison of the XRPD diffraction patterns of the dihydrated form of the racemic compound (monocrystal prepared below in Example 8) versus the crystalline forms A, B, D, E.

Figure 13 shows the XRPD diffraction pattern of Form C.

The characteristic data of these diffraction patterns are as follows.

The characteristic peaks (or lines) are generally those of the highest intensity. The specific peaks (or lines) are specific to that particular polymorphic or pseudopolymorphic form.

| Form A (theoretical XRFD at 233K) | | | 2 Theta (experimental XRPD at 293 K) |
|---|---|---|---|
| Characteristic peaks | Interplanar spacing (Å) | 2 Theta | |
| 1 | 10.39 | 8.50 | 8.48 |
| 2 | 5.76 | 5.36 | 15.34 |
| 3 | 5.40 | 16.41 | 16.38 |
| 4 | 5.19 | 17.05 | 17.04 |
| 5 | 3.66 | 24.30 | 24.28 |
| Specific peak | 10.39 | 8.50 | 8.48 |

| **Form B** | | |
|---|---|---|
| **Characteristic peaks** | **Interplanar spacing (Å)** | **2 Theta (experimental XRPD at 293 K)** |
| 1 | 6.83 | 12.97 ± 0.02 |
| 2 | 5.39 | 16.45 ± 0.02 |
| 3 | 5.14 | 17.24 ± 0.01 |
| 4 | 5.08 | 17.45 ± 0.02 |
| 5 | 3.99 | 22.29 ± 0.02 |

| Specific peaks | | |
|---|---|---|
| 1 | 8.54 | 10.36 ± 0.01 |
| 2 | 6.83 | 12.91 ± 0.02 |

| **Form D** | | |
|---|---|---|
| **Characteristic peaks** | **Interplanar spacing (Å)** | **2 Theta (experimental XRPD at 293 K)** |
| 1 | 5.46 | 16.23 ± 0.02 |
| 2 | 5.09 | 17.44 ± 0.01 |
| 3 | 5.00 | 17.75 ± 0.01 |
| 4 | 4.79 | 18.53 ± 0.01 |
| 5 | 4.00 | 22.22 ± 0.01 |
| Specific peak | 7.12 | 12.43 ± 0.01 |

| **Form E (theoretical XRPD at 296K)** | | | **2 Theta (experimental XRPD at 293 K)** |
|---|---|---|---|
| **Characteristic peaks** | **Interplanar spacing (Å)** | **2 Theta** | |
| 1 | 6.48 | 13.65 | 13.67 |
| 2 | 5.90 | 15,01 | 15.04 |
| 3 | 5.76 | 15.38 | 15.39 |
| 4 | 5.63 | 15.72 | 15.75 |
| 5 | 4.57 | 19.42 | 19.52 |

| **Specific peaks** | | | |
|---|---|---|---|
| 1 | 5.90 | 15.01 | 15.04 |
| 2 | 3.62 | 24.57 | 24.56 |

| **Specific peaks (at 293K)** | | |
|---|---|---|
| **Form** | **Interplanar spacing (Å)** | **2 Theta** |
| A | 10.42 | 8.48 |
| B | 8.54 6.83 | 10.36 ± 0.01 12.97 ± 0.02 |
| D | 7.12 | 12.43 ± 0.01 |
| E | 5.90 | 15.01 |
| | 3.62 | 24.57 |

| **Dihydrate racemic form (theoretical XRPD at 296K)** | | |
|---|---|---|
| **Characteristic peaks** | **Interplanar spacing (Å)** | **2 Theta** |
| 1 | 13.92 | 6.34 |
| 2 | 5.48 | 16.15 |
| 3 | 5.30 | 16.70 |
| 4 | 4.92 | 18.01 |
| 5 | 3.31 | 26.90¹⁰ |

As mentioned above, the azabicyclic compounds as described in Applications WO 02/10172 and WO 03/063864 are very useful in antibacterial therapeutics. This is in particular the case with the sodium salt of 7-oxo-6-sulfooxy-1,6-diazabicyclo[3.2.1]octane-2-carboxamide described in these applications, because of its remarkable inhibitory action on beta-lactamases in pathogenic bacteria.

Owing to their intrinsic properties, the sodium salt of (1R,2S,5R)-7-oxo-6-sulfooxy-1,6-diazabicyclo[3.2.1]octane-2-carboxamide, and in particular the polymorphic and pseudopolymorphic crystalline forms "A", "B", "D" and "E", are particularly suitable for use in antibacterial therapeutics.

The invention thus also relates to said sodium salt in its polymorphic crystalline form "B" for their use as a medicament, and in particular a medicament that is an inhibitor of beta-lactamases.

According to another aspect of the present invention there is provided the polymorphic crystalline form "B" of the sodium salt of (1R,2S,5R)-7-oxo-6-sulfooxy-1,6-diazabicyclo[3.2.1]octane-2-carboxamide as described herein in combination with an antibacterial agent for use in treating bacterial infections.

Antibacterial agents for use in combination with the polymorphic and pseudopolymorphic crystalline forms "A", "B", "D" and "E" of the sodium salt of (1R,2S,5R)-7-oxo-6-sulfooxy-1,6-diazabicyclo[3.2.1]octane-2-carboxamide are preferably antibiotics of the β-lactamine type. Antibiotics of the β-lactamine type include penams, penems, cephems, carbacephems, oxacephems, cephamycins, also penicillins such as amoxicillin, ampicillin, azlocillin, mezlocillin, apalcillin, hetacillin, bacampicillin, carbenicillin, sulbenicillin, ticarcillin, piperacillin, mecillinam, pivmecillinam, methicillin, ciclacillin, talampicillin, aspoxicillin, oxacillin, cloxacillin, dicloxacillin, flucloxacillin, nafcillin or pivampicillin, also cephalosporins such as cephalothin, cephaloridine, cefaclor, cefadroxil, cefamandole, cefazolin, cephalexin, cephradine, ceftizoxime, cefoxitin, cephacetrile, cefotiam, cefotaxime, cefsulodin, cefoperazone, ceftizoxime, cefmenoxime, cefmetazole, cephaloglycin, cefonicid, cefodizime, cefpirome, ceftazidime, ceftaroline or a prodrug thereof such as ceftaroline fosamil, ceftriaxone, cefpiramide, cefbuperazone, cefozopran, cefepime, cefoselis, cefluprenam, cefuzonam, cefpimizole, cefclidin, cefixime, ceftibuten, cefdinir, cefpodoxime axetil, cefpodoxime proxetil, cefteram pivoxil, cefetamet pivoxil, cefcapene pivoxil, or cefditoren, pivoxil, cefuroxime, cefuroxime axetil, loracarbacef or latamoxef, also carbapenems such as imipenem, meropenem, biapenem or panipenem and also monobactams such as aztreonam and carumonam, as well as their salts.

A particular antibacterial agent is ceftazidime.

According to another aspect of the present invention there is provided polymorphic form "Form B", of the sodium salt of (1R,2S,5R)-7-oxo-6-sulfooxy-1,6-diazabicyclo[3.2.1]octane-2-carboxamide as described herein, in combination with ceftazidime for use in the treatment of bacterial infections.

A particular antibacterial agent is ceftaroline fosamil.

According to another aspect of the present invention there is provided the polymorphic form "Form B", of the sodium salt of (1R,2S,5R)-7-oxo-6-sulfooxy-1,6-diazabicyclo[3.2.1]octane-2-carboxamide as described herein, in combination with ceftaroline fosamil for use in treating bacterial infections.

Ceftaroline is a novel parenteral cephalosporin with a broad spectrum of activity against clinically important community-acquired and hospital-acquired Gram-negative and Gram-positive pathogens including methicillin-resistant *Staphylococcus aureus* and multidrug-resistant *Streptococcus pneumoniae.*

U.S. Patent No. 6,417,175 discloses compounds having excellent antibacterial activities for a broad range of Gram-positive and Gram-negative bacteria. These compounds are represented by the general formula: 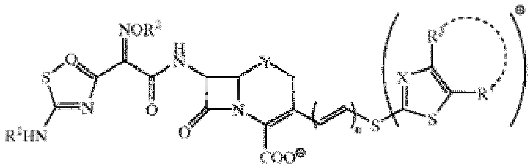 wherein R1-R4, Q, X, Y and n are as defined therein.

U.S. Patent No. 6,417,175 discloses methods for preparing the compounds, and generically discloses formulations of the compounds, such as aqueous and saline solutions for injection. One such compound is 7β-[2(Z)-ethoxyimino-2-(5-phosphonoamino-1,2,4-thiadiazole-3-yl)acetamido]-3-[4-(1-methyl-4-pyridinio)-2-thiazolythio]-3-cephem-4-carboxylate.

U.S. Patent No. 6,906,055 discloses a chemical genus which includes compounds of formula:

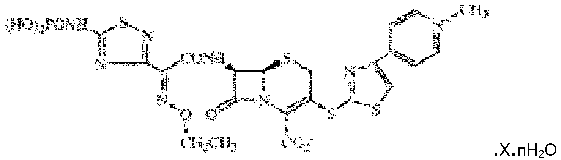

Ceftaroline fosamil is a sterile, synthetic, parenteral prodrug cephalosporin antibiotic. The N-phosphonoamino water-soluble prodrug is rapidly converted into the bioactive ceftaroline, which has been demonstrated to exhibit antibacterial activity. Ceftaroline fosamil is known as (6R,7R)-7-{(2Z)-2-(ethoxyimino)-2-[5-(phosphonoamino)- 1,2,4-thiadiazol-3-yl]acetamido}-3-{[4-(1-methylpyridin-1-ium-4-yl)-1,3-thiazol-2-yl]sulfanyl}-8-oxo-5-thia-1-azabicyclo[4.2.0]oct-2-ene-2-carboxylate.

Ceftaroline fosamil may be an acetic acid hydrous form.

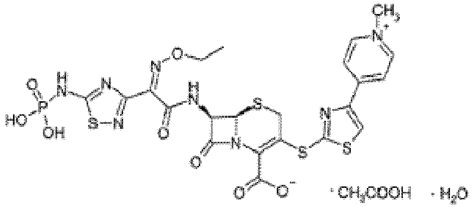

U.S. Patent No. 7,419,973 discloses compositions comprising ceftaroline fosamil and a pH adjuster, such as, L-arginine.

The bacterial infections include, but are not limited to complicated skin and structure infection and community acquired pneumonia. In some embodiments, the community acquired pneumonia may be due to a microorganism, such as, *Streptococcus, Staphylococcus, Haemophilus, Klebsiella, Escherichia* and *Moraxella.* In further embodiments, the community acquired bacterial pneumonia may be due to a microorganism, including, but not limited to, *Streptococcus pneumoniae, Staphylococcus aureus, Haemophilus influenzae, Haemophilus parainfluenzae, Klebsiella pneumoniae, Escherichia coli and Moraxella catarrhalis.* In other embodiments, the community acquired pneumonia may be due to *Enterobacter, Proteus* or *Serratia.* In further embodiments, the community acquired bacterial pneumonia may be due to *Enterobacter aerogenes, Proteus mirabilis* or *Serratia marcescens.*

In exemplary embodiments, the microorganism may be *Streptococcus pneumoniae.* The strain of *Streptococcus pneumoniae* may be penicillin-susceptible, penicillin-resistant or multidrug resistant. In exemplary embodiments, the microorganism may be *Streptococcus pneumoniae* serotype 19A. In some embodiments, the community acquired pneumonia may be associated with concurrent bacteremia. In other exemplary embodiments, the microorganism may be *Staphylococcus aureus.* The strain or isolate of *Staphylococcus aureus* may be methicillin-susceptible or methicillin-resistant. In still other exemplary embodiments, the microorganism may be *Haemophilus influenzae, Klebsiella pneumoniae* or *Escherichia coli.* In exemplary embodiments, the microorganism may be a β-lactamase-nonproducing ampicillin-resistant (BLNAR) strain of *Haemophilus influenzae.*

The medicaments as defined above are utilized in the form of pharmaceutical compositions, if necessary mixed with a pharmaceutically acceptable organic or mineral excipient, suitable for the sought method of administration, and the invention also relates to said pharmaceutical compositions.

The crystalline forms "B" of the present invention can be presented for administration to patients alone or in combination with an antibacterial agent, such as, for example, ceftazidime, ceftaroline or a prodrug of ceftaroline such as ceftaroline fosamil. The present invention includes pharmaceutical compositions comprising the crystalline form of the invention alone or in combination with an antibacterial agent, such as, for example, ceftazidime, ceftaroline or a prodrug of ceftaroline such as ceftaroline fosamil. The compositions may further comprise one or more pharmaceutically acceptable carriers.

These compositions can be solid or liquid and are presented in the pharmaceutical forms commonly used in human medicine such as, for example, plain or coated tablets, capsules, granules, suppositories, injectable preparations, ointments, creams, gels; they are prepared by the usual methods. The active ingredient or ingredients can be incorporated in them with excipients usually utilized in these pharmaceutical compositions, such as talc, gum arabic, lactose, starch, magnesium stearate, cocoa butter, aqueous or non-aqueous vehicles, fats of animal or vegetable origin, paraffin derivatives, glycols, various wetting agents, dispersants or emulsifiers and preservatives.

These compositions can also be in the form of sodium salt according to the invention, and in particular the polymorphic form "B" as such, intended to be dissolved extemporaneously in a suitable vehicle, for example apyrogenic sterile water.

The invention finally relates to the pharmaceutical compositions as defined above, characterized in that they further contain, as ingredient, an antibacterial medicament of the beta-lactamine type.

The crystalline form of the present invention can be used to treat a patient at the same time as the dose of an antibacterial agent, or separately. In exemplary embodiments, the crystalline form may be used in combination with the antibacterial agent, e.g. ceftazidime or ceftaroline fosamil in one composition. In other embodiments, a composition comprising the crystalline form may be used to treat a patient concurrently with a composition comprising the antibacterial agent (e.g. ceftazidime or ceftaroline fosamil).

The dose of the crystalline form "B" may vary according to several factors, including, but not limited to the type of bacterial infection and the microorganism causing the infection.

In some embodiments, the daily dose of the crystalline form may range from about 0.1 to approximately about 10 g. In specific embodiments, the daily dose of the crystalline form may be about 100 mg to 10 g. In other embodiments, the daily dose of the crystalline form may be about 200 mg to 5 g. In still other embodiments, the daily dose of the crystalline form may be about 200 mg to 2000 mg. In exemplary embodiments, the daily dose of the crystalline form may be about 200 mg, about 300 mg, about 400 mg, about 500 mg, about 600 mg, about 700 mg, about 800 mg, about 900 mg, about 1000 mg, about 1 100 mg, about 1200 mg, about 1300 mg, about 1400 mg, about 1500 mg, about 1600 mg, about 1700 mg, about 1800 mg, about 1900 mg and about 2000 mg. In some exemplary embodiments, the daily dose is 800 mg. In other exemplary embodiments, the daily dose is 1200 mg. In some exemplary embodiments, the daily dose is 800 mg. In other exemplary embodiments, the daily dose is 500 mg.

Some embodiments comprise administering the crystalline form in combination with between about 100 mg and about 2400 mg of ceftazidime. In further embodiments, ceftazidime may be administered in an amount between about 100 mg and about 1200 mg. In some embodiments, ceftazidime may be administered in an amount between about 200 mg and 1000 mg. In exemplary embodiments, the amount may be about 100 mg, 200 mg, 300 mg, 400 mg, 500 mg, 600 mg, 700 mg, 800 mg, 900 mg, 1000 mg, 1 100 mg or 1200 mg. In certain embodiments, the amount may be about 400 mg. In other embodiments, the amount may be about 600 mg. In still other embodiments, the amount may be about 800 mg. In certain embodiments, the amount may be about 1200 mg. In certain embodiments, the amount of ceftazidime may be about 2000 mg.

Some embodiments comprise administering the crystalline form in combination with between about 100 mg and about 2400 mg of ceftaroline or a prodrug thereof (e.g. ceftaroline fosamil). In further embodiments, ceftaroline or a prodrug thereof may be administered in an amount between about 100 mg and about 1200 mg. In some embodiments, ceftaroline or a prodrug thereof may be administered in an amount between about 200 mg and 1000 mg. In exemplary embodiments, the amount may be about 100 mg, 200 mg, 300 mg, 400 mg, 500 mg, 600 mg, 700 mg, 800 mg, 900 mg, 1000 mg, 1 100 mg or 1200 mg. In certain embodiments, the amount may be about 400 mg. In other embodiments, the amount may be about 600 mg. In still other embodiments, the amount may be about 800 mg. In certain embodiments, the amount of ceftaroline fosamil may be about 1200 mg.

The amount of the crystalline form and antibacterial agent may be used to provide a single dose or multiple divided doses per day. For example, the amount may be used as a single daily dose. In exemplary embodiments, about 800 mg of the crystalline form "B" may be administered daily with about 800 mg of ceftaroline or a prodrug thereof (e.g., ceftaroline fosamil). In other exemplary embodiments, about 1200 mg of the crystalline form "B" may be administered daily with about 1200 mg of ceftaroline or a prodrug (e.g., ceftaroline fosamil) thereof. In some embodiments, the amount may be administered in two to eight doses per day. For example, about 400 mg of the crystalline form and about 400 mg of ceftaroline or a prodrug thereof (e.g., ceftaroline fosamil) may be administered every 12 hours (i.e. twice a day). In some examples, about 600 mg of the crystalline form and about 600 mg of ceftaroline or a prodrug thereof (e.g., ceftaroline fosamil) may be administered every 12 hours (i.e. twice a day).

In some embodiments, the ratio of the crystalline form to the antibacterial agent may range from about 1:20 to about 10:1. The ratio may vary according to the type of infection and the antibacterial agent. In exemplary embodiments, the ratio of crystalline form to antibacterial agent may be between about 1:10 to 5:1.

In specific embodiments, the methods comprise administering the crystalline form in combination with ceftaroline or a prodrug of ceftaroline, such as, ceftaroline fosamil. In exemplary embodiments, the methods include administering the crystalline form and ceftaroline fosamil in a ratio of about 1:1 to 5:1 , such as, for example, 1:1, 2:1, 3:1, 4:1, 5:1. Exemplary embodiments comprise administering the crystalline form "B" and ceftaroline fosamil in a ratio of 1:1. For example, about 400 mg of Form "B" may be administered in combination with about 400 mg of ceftaroline fosamil. In some embodiments, about 600 mg of Form "B" may be administered with about 600 mg of ceftaroline fosamil.

In exemplary embodiments, the crystalline form and ceftaroline or a prodrug thereof may be administered parenterally. Suitable methods for parenteral administration include, but are not limited to, administering a sterile aqueous preparation of the crystalline form alone or in combination with an antibacterial agent, which preferably is isotonic with the blood of the recipient (e.g., physiological saline solution). Such preparations may include suspending agents and thickening agents and liposomes or other microparticulate systems, which are designed to target the compound to blood components or one or more organs. The preparation may be presented in a unit-dose or multi-dose form.

Unless defined otherwise, all technical and scientific terms used herein generally have the same meaning as commonly understood by one of ordinary skill in the art to which this invention belongs.

The term "prodrug" means a compound that is a drug precursor, which upon administration to a subject undergoes chemical conversion by metabolic or chemical processes to yield a compound, which is an active moiety. Suitable prodrugs of ceftaroline include, but are not limited to, phosphonocephem derivatives, such as, e.g. 7β-[2(Z)-ethoxyimino-2-(5-phosphonoamino-1,2,4-thiadiazol-3-yl)acetamido]-3-[4-(1-methyl-4-pyridinio)-2-thiazolythio]-3-cephem-4-carboxylate.

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per practice in the art. Alternatively, "about" with respect to the compositions can mean plus or minus a range of up to 20%, preferably up to 10%, more preferably up to 5%. Alternatively, particularly with respect to biological systems or processes, the term can mean within an order of magnitude, preferably within 5 -fold, and more preferably within 2-fold, of a value. Where particular values are described in the application and claims, unless otherwise stated the term "about" means within an acceptable error range for the particular value. For example, when referring to a period of time, e.g., hours, the present values (± 20%) are more applicable. Thus, 6 hours can be, e.g., 4.8 hours, 5.5 hours, 6.5 hours, 7.2 hours, as well as the usual 6 hours.

The terms "treat," "treatment," and "treating" refer to one or more of the following: relieving or alleviating at least one symptom of a bacterial infection in a subject; relieving or alleviating the intensity and/or duration of a manifestation of bacterial infection experienced by a subject; and arresting, delaying the onset (i.e., the period prior to clinical manifestation of infection) and/or reducing the risk of developing or worsening a bacterial infection.

The term "community acquired pneumonia" as used herein is equivalent and has been used interchangeably with the term "community acquired bacterial pneumonia."

The term "therapeutically effective" applied to dose or amount refers to that quantity of a compound or pharmaceutical composition that is sufficient to result in a desired activity upon administration to a mammal in need thereof. An "effective amount" means the amount of a compound according to the invention that, when administered to a patient for treating an infection or disease is sufficient to effect such treatment. The "effective amount" will vary depending on the active ingredient, the state of infection, disease or condition to be treated and its severity, and the age, weight, physical condition and responsiveness of the mammal to be treated.

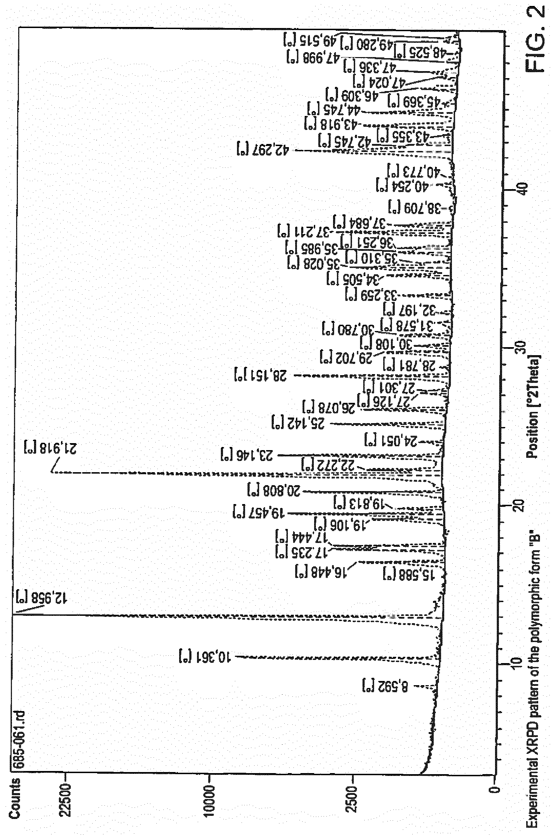

It is known that an X-ray powder diffraction pattern may be obtained which has one or more measurement errors depending on measurement conditions (such as equipment or machine used). In particular, it is generally known that intensities in an X-ray powder diffraction pattern may fluctuate depending on measurement conditions. Therefore it should be understood that the crystalline form B of the present invention is not limited to the crystals that provide X-ray powder diffraction pattern identical to the X-ray powder diffraction pattern shown in Figure 2 and any crystals providing X-ray powder diffraction patterns substantially the same as those shown in Figure 2 fall within the scope of the present invention. A person skilled in the art of X-ray powder diffraction is able to judge the substantial identity of X-ray powder diffraction patterns.

Persons skilled in the art of X-ray powder diffraction will realise that the relative intensity of peaks can be affected by, for example, grains above 30 microns in size and non-unitary aspect ratios, which may affect analysis of samples. The skilled person will also realise that the position of reflections can be affected by the precise height at which the sample sits in the diffractometer and the zero calibration of the diffractometer. The surface planarity of the sample may also have a small effect. Hence the diffraction pattern data presented are not to be taken as absolute values. (Jenkins, R & Snyder, R.L. 'Introduction to X-Ray Powder Diffractometry' John Wiley & Sons 1996; Bunn, C.W. (1948), Chemical Crystallography, Clarendon Press, London; Klug, H. P. & Alexander, L. E. (1974), X-Ray Diffraction Procedures).

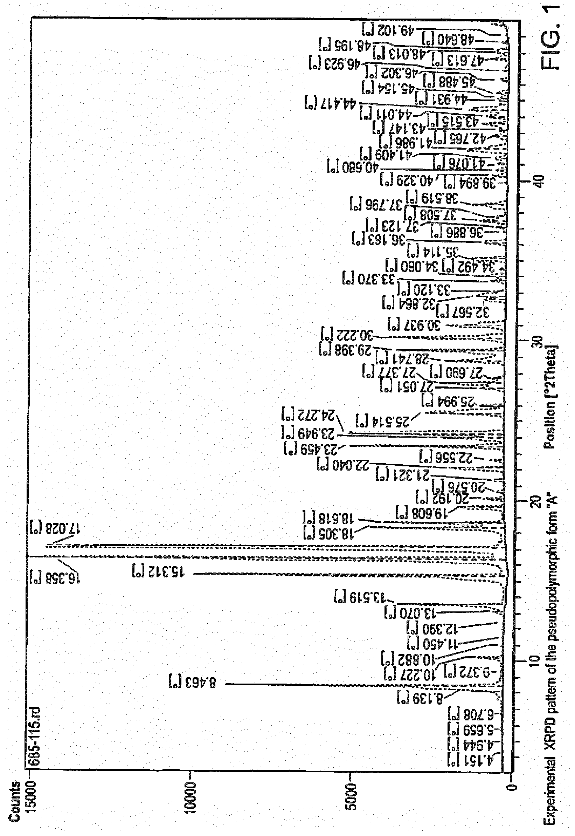

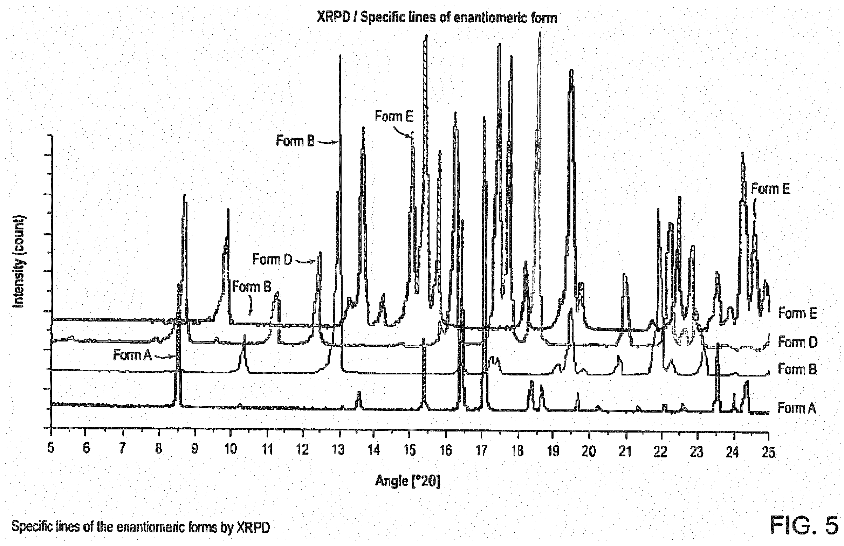

Generally, a measurement error of a diffraction angle in an X-ray powder diffractogram is about 5% or less, in particular plus or minus 0.5° 2-theta, and such degree of a measurement error should be taken into account when considering the X-ray powder diffraction patterns in Figures 1 to 10, 12 and 13. Furthermore, it should be understood that intensities may fluctuate depending on experimental conditions and sample preparation (preferred orientation).

The invention is illustrated by the following examples:

### Example 1: Sodium salt of the (1R,2S,5R)-7-oxo-6-sulfooxy-1,6-diazabicyclo[3.2.11octane-2-carboxamide enantiomer - polymorphic form "B"

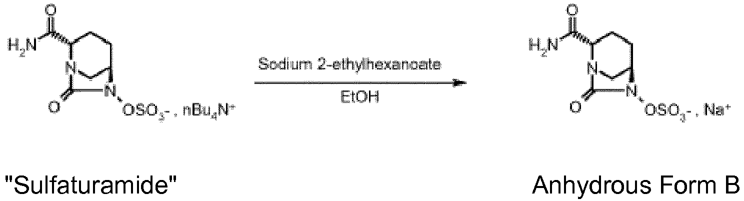

A solution of sodium 2-ethylhexanoate (13.12 g, 79 mmol) in ethanol (126 mL) is added over five hours to a solution of sulfaturamide (20 g, 39.5 mmol) in ethanol (126 mL) stirred at 30°C and seeded with a few crystals of polymorphic form "B". The suspension is stirred overnight. The suspension is cooled to 0-5°C for 1 to 2 hours, filtered and then washed with ethanol at 5°C (3x40 mL). The crystals are dried under reduced pressure of 20 mbar at 20°C. The polymorphic form "B" is obtained (10.79 g, 37.5 mmol, yield 95.1%).

An X-ray spectrum ("XRPD diffraction pattern") was recorded and is shown in Figure 2.

### Example 2: Sodium salt of the (1R,2S,5R)-7-oxo-6-sulfooxy-1,6-diazabicyclo[3.2.1]octane-2-carboxamide enantiomer - polymorphic form "B"

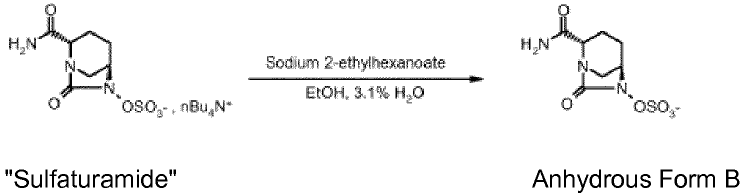

A solution of sulfaturamide (10 g, 19.7 mmol) in ethanol (100 ml) is added over forty-five minutes to a solution of sodium 2-ethylhexanoate (3.80 g, 22.9 mmol) in ethanol (95 ml) and water (5 ml; 3.1% of the total weight of the solvent), stirred at room temperature and seeded with a few crystals of pseudopolymorphic form "A". The suspension is stirred overnight. The suspension is cooled down to 0-5°C for 1 to 2 hours, filtered and then washed with ethanol at 5°C (3x30 ml). The crystals are dried under reduced pressure of 20 mbar at 20°C. The polymorphic form "B" is obtained (4.277 g, 14.9 mmol, yield 75.4%).
Water (Karl Fischer): 0.2%
DSC: Exothermic peak at 221.9°C
The XRPD spectrum obtained corresponds to form B

### Reference Example 3: Sodium salt of the (1R,2S,5R)-7-oxo-6-(sulfooxy)-1,6-diazabicyclo[3.2.1]octane-2-carboxamide enantiomer, monohydrate - pseudopolymorphic form "A"

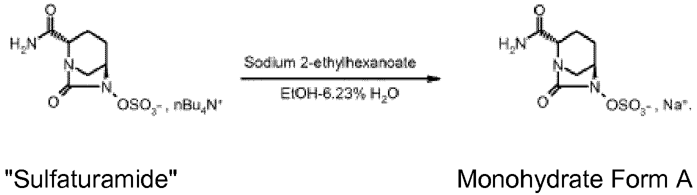

A solution of sodium 2-ethylhexanoate (6.56 g, 39.4 mmol) in ethanol (70 mL) is added over forty-five minutes to a solution of sulfaturamide (10 g, 19.7 mmol) in a mixture of ethanol (63 mL) and water (7 mL, 6.23% of the total weight of the solvent), stirred at 20°C and seeded with the pseudopolymorphic form "A". The suspension is stirred overnight. The suspension is cooled down to 0-5°C for 1 to 2 hours, filtered and then washed with aqueous ethanol (5%) cooled down to 5°C (3x20 mL). The crystals are dried under reduced pressure of 20 mbar at 20°C. The pseudopolymorphic form A is obtained (5.35 g, 17.5 mmol, yield 88.8%).

An X-ray spectrum ("XRPD diffraction pattern") was recorded and is shown in Figure 1.

### Reference Example 4: Sodium salt of the (1R,2S,5R)-7-oxo-6-(sulfooxy)-1,6-diazabicyclo[3.2.1]octane-2-carboxamide enantiomer, monohydrate - pseudopolymorphic form "A"

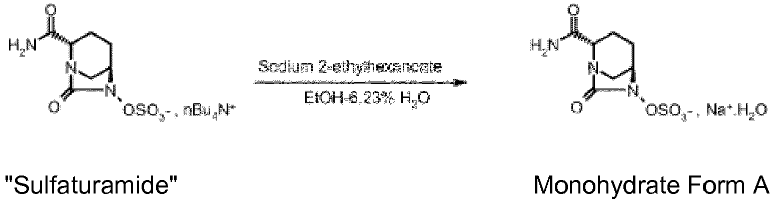

A solution of sulfaturamide (1 g, 1.97 mmol) in ethanol (9.5 ml) and water (0.5 ml) is added over thirty minutes to a solution of sodium 2-ethylhexanoate (0.506 g, 3.04 mmol) in ethanol (9.5 ml) and water (0.5 ml). It is stirred at room temperature. The solution (6.23% of the total weight of water) is seeded with a few crystals of pseudopolymorphic form "A" to produce a suspension, which is stirred overnight. The suspension is cooled down to 0-5°C for 1 to 2 hours, filtered and then washed with ethanol at 5°C (3x6 ml). The crystals are dried under reduced pressure of 20 mbar at 20°C. The pseudopolymorphic form "A" is obtained (0.378 g, 1.24 mmol, yield 62.7%).
Water (Karl Fischer): 5.72% (theoretical 5.9%)
DSC: Exothermic peak at 238.9°C
The XRPD spectrum obtained corresponds to form A

### Reference Example 5: Sodium salt of the (1R,2S,5R)-7-oxo-6-sulfooxy-1,6-diazabicyclo[3.2.1]octane-2-carboxamide enantiomer - polymorphic form "D"

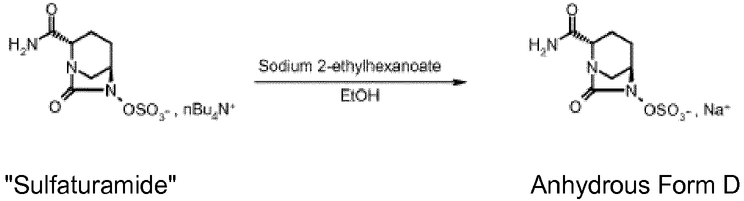

A solution of sodium 2-ethylhexanoate (3.28 g, 19.7 mmol) in ethanol (25 ml) is added over thirty minutes to a solution of sulfaturamide (4 g, 9.87 mmol) in ethanol (25 ml), stirred at 20°C and seeded with the polymorphic form "A". The suspension is stirred overnight. The suspension is filtered and then washed with ethanol at 5°C (3x10 ml). The solid is dried under reduced pressure of 20 mbar at 20°C. The polymorphic form "D" is obtained (2.50 g, 8.70 mmol, yield 88.2%).

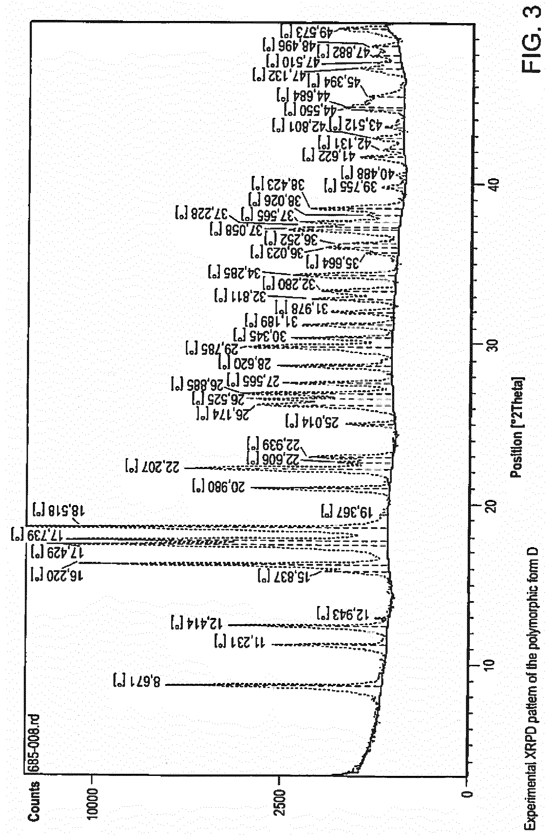

An X-ray spectrum ("XRPD diffraction pattern") was recorded and is shown in Figure 3.

### Reference Example 6: Sodium salt of the (1R,2S,5R)-7-oxo-6-(sulfooxy)-1,6-diazabicyclo[3.2.1]octane-2-carboxamide enantiomer, dehydrate - pseudopolymorphic form "E"

A sample of sodium salt of the (1R,2S,5R)-7-oxo-6-(sulfooxy)-1,6-diazabicyclo[3.2.1]octane-2-carboxamide enantiomer, monohydrate - pseudopolymorphic form "A" (1 g) is suspended in water (2 ml). The suspension, unstirred, is left to evaporate slowly at ambient temperature, pressure and humidity.

The crystallized solid is recovered after complete evaporation. The pseudopolymorphic form "E" is obtained (1.056 g).

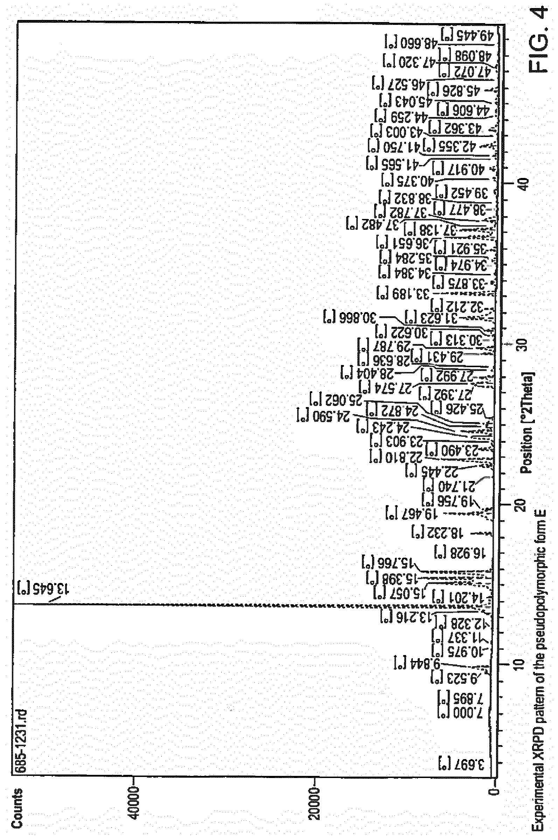

An X-ray spectrum ("XRPD diffraction pattern") was recorded and is shown in Figure 4.

### Reference Example 7: Sodium salt of racemic trans-7-oxo-6-(sulfooxy)-1,6-diazabicyclo[3.2.1]octane-2-carboxamide

A solution, in a water-acetone mixture (1-1), of the sodium salt of the racemic trans-7-oxo-6-(sulfooxy)-1,6-diazabicyclo[3.2.1]octane-2-carboxamide described in Example 33c of Application WO 02/10172 is evaporated under reduced pressure, under the conditions of concentration described in said example. The salt is indeed obtained in crystallized form as stated in the application.

An X-ray spectrum ("XRPD diffraction pattern") was recorded and is shown in Figure 6.

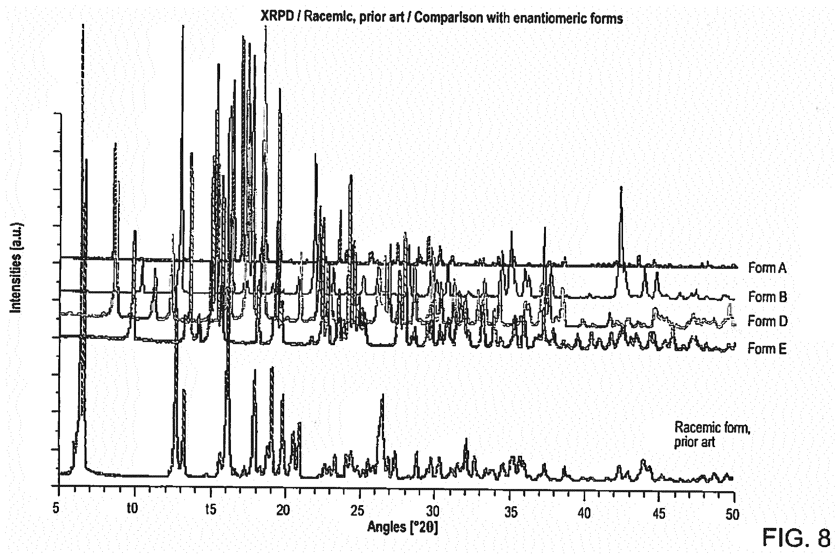

The X-ray spectra ("XRPD diffraction patterns") of the polymorphic and pseudopolymorphic forms A, B, D and E of the enantiomer were compared. The diffraction pattern of the racemic form obtained according to the prior art is different from each of those of the polymorphic and pseudopolymorphic crystallized forms of the enantiomer described herein, as is clearly apparent in Figure 8 in the attached drawings. The characteristic lines of the polymorphic and pseudopolymorphic forms "A", "B", "D" or "E" do not occur in the XRPD diffraction pattern of the racemic compound of the prior art.

The diffraction pattern of the racemic form obtained by the prior art is a mixture of several forms including the dihydrate racemic form.

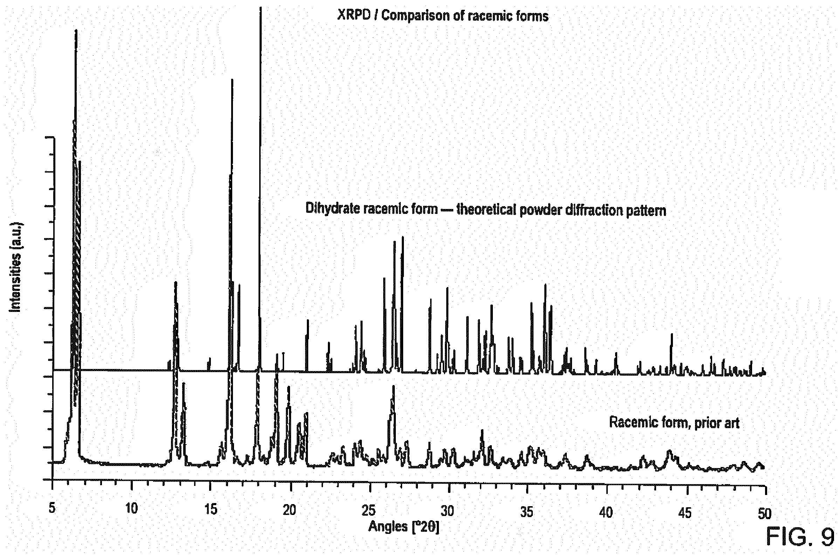

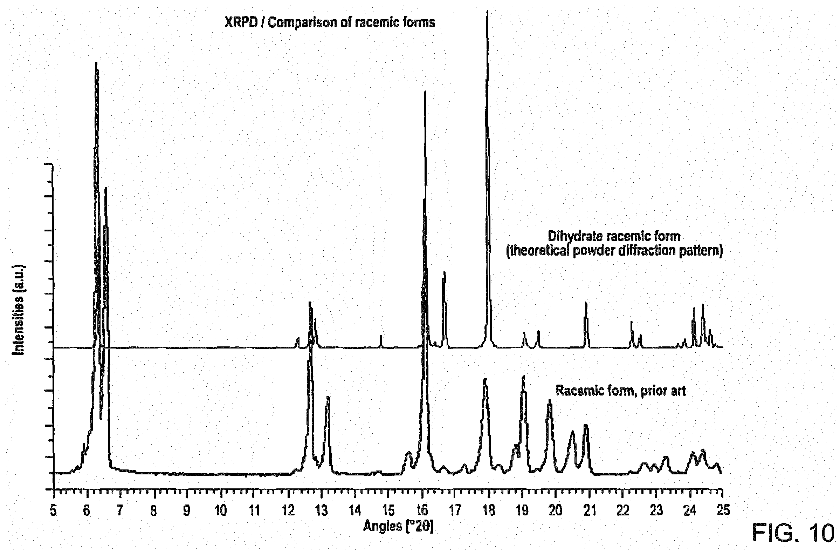

Figs. 9 and 10 in the attached drawings show the XRPD diffraction patterns of the racemic compound.

### Reference Example 8: Preparation of a monocrystal of sodium salt of racemic trans-7-oxo-6-(sulfooxy)-1,6-diazabicyclo[3.2.1]octane-2-carboxamide for structural analysis

A solution of the sodium salt of the racemic trans-7-oxo-6-(sulfooxy)-1,6-diazabicyclo[3.2.1]octane-2-carboxamide described in Example 33c of Application WO 02/10172 and obtained in Example 7 above (50 mg) in a mixture of water (0.5 mL, 10 volumes) and acetone (0.5 mL, 10 volumes) is deposited on a watch glass under an inverted open beaker. After slow evaporation, the crystals are partially dissolved in a mixture of water (1 mL, 20 volumes) and acetone (1 mL, 20 volumes). After the second evaporation, a monocrystal of sufficient size is obtained for structural analysis.

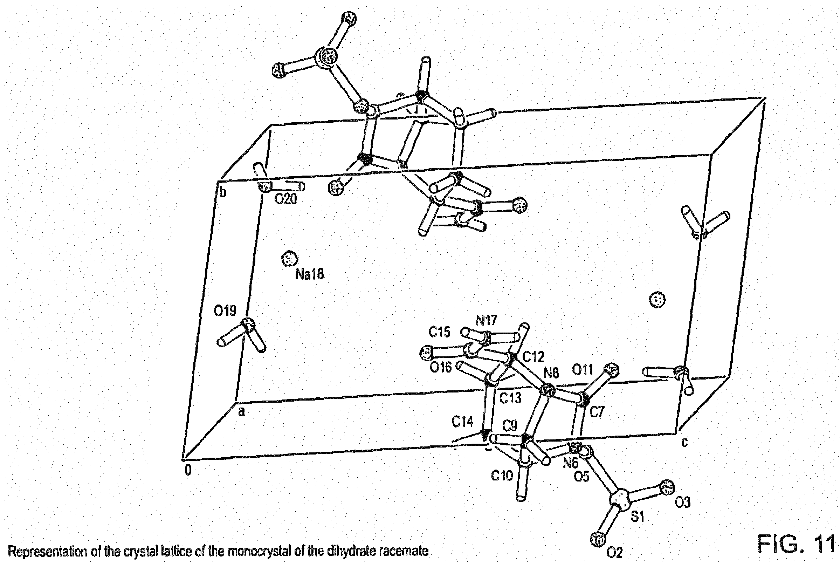

Analysis of the monocrystal of this material, as presented in Figure 11, shows the presence of both enantiomers and two molecules of water within the unit cell, characteristic of a dihydrated racemic compound.

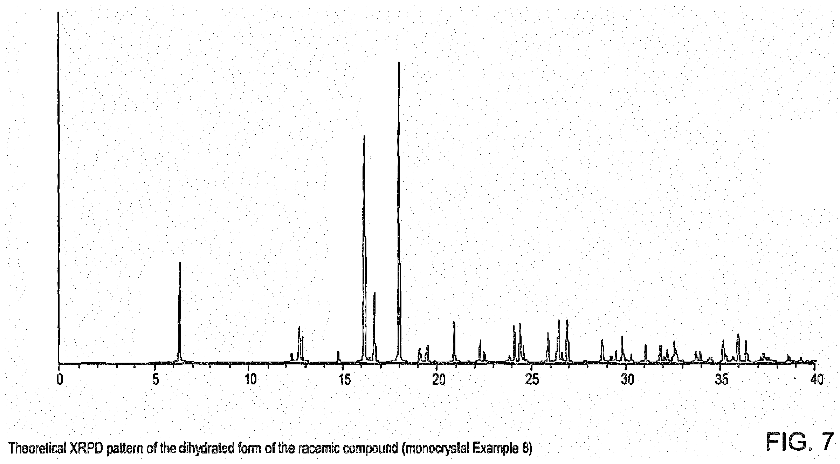

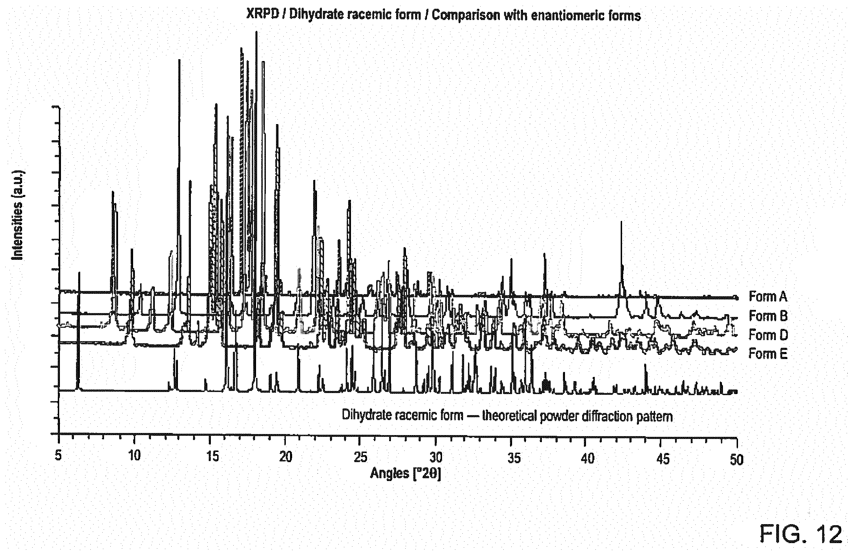

A theoretical XRPD spectrum was calculated on the basis of the monocrystal and is shown in Figure 7. The X-ray spectra ("XRPD diffraction patterns") of the monocrystal and of the polymorphic and pseudopolymorphic forms A, B, D and E of the enantiomer were compared. The diffraction pattern of the monocrystal of the racemic salt is different from each of those of the polymorphic and pseudopolymorphic crystallized forms of the enantiomer described herein, as is clearly apparent in Figure 12. The characteristic lines of the polymorphic and pseudopolymorphic forms "A", "B", "D" or "E" do not occur in the XRPD diffraction pattern of the racemic dihydrate compound.

### Example 9: Preparation of "Sulfaturamide" or tetrabutylammonium salt of (1R,2S,5R)-7-oxo-6-(sulfooxy)-1,6-diazabicyclo[3.2.1]octane-2-carboxamide

Method A: The "sulfaturamide" compound can be prepared by chiral resolution of its racemic precursor trans-7-oxo-6-(phenylmethoxy)-1,6-diazabicyclo[3.2.1]octane-2-carboxamide, the preparation of which is described in Example 33a Stage A in Application WO 02/10172.

Injection of 20 µl of a sample of 0.4 mg/mL of trans-7-oxo-6-(sulfooxy)-1,6-diazabicyclo[3.2.1]octane-2-carboxamide, eluted on a Chiralpak ADH column (5 µm, 25 cmx4.6 mm) with heptane-ethanol-diethylamine mobile phase 650/350/0.05 vol at 1 mL/min makes it possible to separate the (1R,2S,5R) and (1S,2R,5S) enantiomers with retention times of 17.4 minutes and 10.8 minutes respectively.

The sulfaturamide is then obtained by conversion according to the conditions described in Example 33a Stage B then Stage C and finally in Example 33b of Application WO 02/10172.

Method B: The "sulfaturamide" can also be prepared from the mixture of the oxalate salt of (2S)-5-benzyloxyamino-piperidine-2-carboxylic acid, benzyl ester (mixture (2S,5R)/(2S,5S) ∼ 50/50) described in application FR2921060.

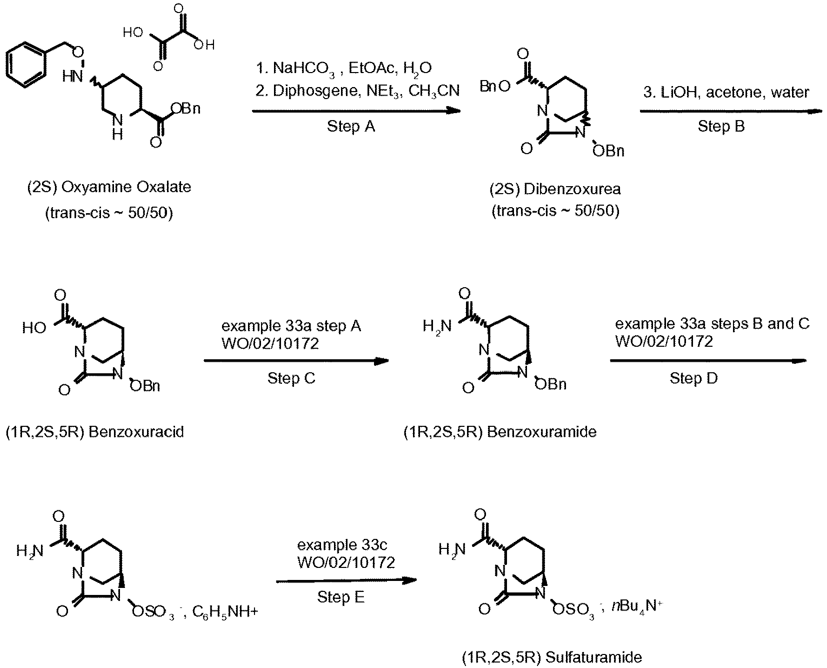

### Stage A: Dibenzoxurea or (2S)-7-oxo-6-(2-phenylmethoxy)-1,6-diaza-bicyclo[3.2.1] octane 2-benzyl 2-carboxylate

A 10% saturated aqueous solution of sodium bicarbonate (16 L) is added to a suspension of the oxalate salt of (2S)-5-benzyloxyamino-piperidine-2-carboxylic acid, benzyl ester (mixture (2S,5R)/(2S,5S) ∼ 50/50) described in application FR2921060 (2 kg, 4.65 mol) in water (12 L) and ethyl acetate (10 L). The aqueous phase is separated and then re-extracted with ethyl acetate (8 L). The organic phases are combined, washed with water (4 L) and then dried over sodium sulfate (2 kg). The solution is filtered and then concentrated in order to replace the ethyl acetate with acetonitrile (35 L). The solution is cooled to 0-5°C before adding triethylamine (1.25 L) and then diphosgene (290 mL). The reaction mixture is stirred at 0-5°C for one hour before adding N,N-dimethylaminopyridine (270 g). After stirring for two hours at room temperature, the reaction mixture is concentrated and then diluted with dichloromethane (15 L). The solution is added to a 20% aqueous solution of ammonium chloride (15 L). The organic phase is isolated. The aqueous phase is re-extracted with dichloromethane (4 L). The organic phases are combined, dried over sodium sulfate and concentrated to dryness to produce the compound (1645 g, yield 96% as is, weight/weight).

### Stage B: Benzoxuracid or (1R,2S,5R)-7-oxo-6-(phenylmethoxy)-1,6-diazabicyclo[3.2.1]octane-2-carboxylic acid and its cyclohexylamine salt.

A solution of lithium hydroxide (79.2 g, 3.3 mol) in water (3.3 L) is added in 30 minutes to a stirred solution at 0-5°C of the compound obtained in Stage A (1.028 kg, 2.80 mol) in water (10.3 L) and tetrahydrofuran (1.5 L). The reaction mixture is stirred for 1.5 h before adding a mixture of isopropyl ether-ethyl acetate (8/2 vol/vol, 9.25 L). The aqueous phase is isolated at room temperature. The organic phase is extracted with water (2x2.57 L). The aqueous phases are combined and then washed with a mixture of isopropyl ether-ethyl acetate (8/2 vol/vol, 2 L). The aqueous solution is stirred with ethyl acetate (10.3 L), acidified with 2N hydrochloric acid (1.9 L) to pH 2 and then saturated with sodium chloride (4.8 kg). The aqueous phase is isolated and re-extracted with ethyl acetate (5.14 L). The organic phases are combined and dried over sodium sulfate (1 kg). The solution is concentrated under vacuum at 40°C to produce the compound (473 g, 61% yield as is, weight/weight).

The cyclohexylamine salt is prepared according to the method described in Example 32b of Application WO 02/10172.

### Stage C: Benzoxuramide or (1R,2S,5R)-7-oxo-6-(phenylmethoxy)-1,6-diazabicyclo[3.2.1]octane-2-carboxamide

This operation is carried out under the conditions described in Example 33a Stage A of Application WO 02/10172 starting with the compound obtained in Stage B above to obtain the compound.

### Stages D and E: "Sulfaturamide"

This operation is carried out starting with the compound obtained in Stage C above, under the conditions described in Example 33a Stage B and then Stage C and finally in Example 33b of Application WO 02/10172. The compound is obtained in solid form.

Reference Example 10: Sodium salt of the amorphous (1R,2S,5R)-7-oxo-6-(sulfooxy)-1,6-diazabicyclo[3.2.1]octane-2-carboxamide enantiomer

A solution of Sulfaturamide (6.92 kg, 13.66 mol) in water (56 L) is eluted on a column of Dowex 50WX8 resin (83 kg, 100-200 mesh) preconditioned by elution of an aqueous solution of sodium hydroxide and then washing with water until a neutral pH is reached. The fractions containing the product are combined, filtered, weighed (76 kg net) and then lyophilized to produce the sodium salt in amorphous form (3.72 kg, yield 94.8%, HPLC purity >99%).

### Reference Example 11: Sodium salt of the (1R,2S,5R)-7-oxo-6-(sulfooxy)-1,6-diazabicyclo[3.2.1]octane-2-carboxamide enantiomer, monohydrate - pseudopolymorphic form "A"

A solution of the 10.134g (20 mmoles) of the tetrabutylammonium salt of (1R,2S,5R)-7-oxo-6-(sulfooxy)-1,6-diazabicyclo[3.2.1]octane-2-carboxamide in 48.1 ml of isobutanol and 2.53 ml water was filtered through a 1.6 µm filter and added to a 500 ml jacketed-reactor equipped with overhead stirrer and internal temperature probe. The solution was warmed to an internal temperature of 35°C. A solution of 6.65 g (40 mmoles) of sodium 2-ethylhexanoate in 49.5 ml isobutanol and 0.5 ml water was filtered through a 1.6 µm filter and added dropwise to the reactor. Crystallization occurred during the addition. The mixture was stirred for an additional 1h at 35°C followed by 16h at 25°C. The mixture was cooled to 0°C for 2h. The crystals were isolated by filtration and washed with an ice-cold mixture of 19.5 ml isobutanol and 0.5 ml water. The crystals were dried under vacuum at 35°C for 20 h. 5.48g of the sodium salt of trans-7-oxo-6-(sulfooxy)-1,6-diazabicyclo[3.2.1]octane-2-carboxamide monohydrate (Form A) was obtained, corresponding to a yield of 90%.

### Reference Example 12: Sodium salt of the (1R,2S,5R)-7-oxo-6-(sulphoxy)-1,6-diazabicyclo[3.2.1]octane-2-carboxamide enantiomer, monohydrate - pseudopolymorphic form "A"

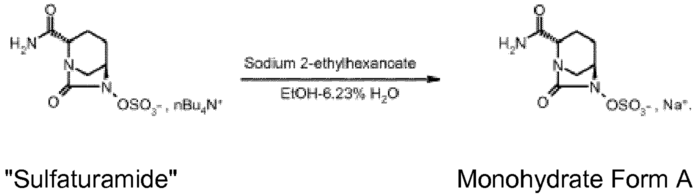

A 5% fraction of a solution of sodium 2-ethyl hexanoate (6.56 g, 39.4 mmol) in ethanol (63 mL) is added over five minutes to a solution of sulfaturamide (10 g, 19.7 mmol) in a mixture of ethanol (63 mL) and water (7 mL, 6.55% of the total weight of the solvent) stirred at 20°C. The mixture is stirred for 15 minutes. The 5% fractional addition/stirring cycle is repeated until spontaneous crystallisation occurs. The suspension is then warmed to 30°C, stirred for one hour then cooled to 20°C. The addition of sodium 2-ethyl hexanoate solution is resumed and completed over forty five minutes. The suspension is cooled down to 0-5°C for 2 hours, filtered and then washed with aqueous ethanol (5%) cooled down to 5°C (2x20 mL). The crystals are dried under reduced pressure of 20 mbar at 30°C. The pseudopolymorphic form A is obtained (5.15 g, 16.9 mmol, yield 85.6%).

An X-ray spectrum ("XRPD diffraction pattern") was recorded and is shown in Figure 1.
Water (Karl Fischer): 6% (theoretical 5.9%)

## Claims

1. The sodium salt of (1R,2S,5R)-7-oxo-6-sulfooxy-1,6-diazabicyclo[3.2.1]octane-2-carboxamide of formula (I) 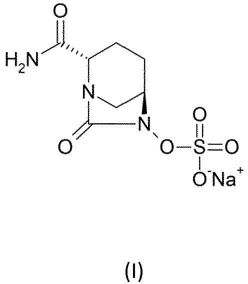 in crystallised form, anhydrous, **characterized in that** the salt is in its polymorphic form "B", having an X-ray diffraction pattern with at least five characteristic peaks at 13.0 +/- 0.5 degrees 2θ, at 16.5 +/- 0.5 degrees 2θ, at 17.2 +/- 0.5 degrees 2θ, at 17.5 +/- 0.5 degrees 2θ, at 22.3 +/- 0.5 degrees 2θ and two specific peaks at 10.4 +/- 0.5 degrees 2θ and at 13.0 +/- 0.5 degrees 2θ.

2. A pharmaceutical composition, **characterized in that** it contains, as ingredient, the sodium salt in crystallised form according to claims 1 and, if appropriate, a pharmaceutically acceptable excipient.

3. A pharmaceutical composition according to claim 2, **characterized in that** it further contains, as ingredient, an antibacterial medicament of the beta-lactamine type.

4. Method for the preparation of the sodium salt according to claim 1, **characterized in that** a solution of sodium 2-ethylhexanoate in pure ethanol is added to a solution of the tetrabutylammonium salt of (1R,2S,5R)-7-oxo-6-sulfooxy-1,6-diazabicyclo[3.2.1]octane-2-carboxamide, in an ethanol/water mixture in such a way that the final proportion of water is from 0 to 5 wt.% of the solvent, operating at a temperature of from 10 to 40°C, in the presence of seed crystals of the polymorphic form "B" or of the pseudopolymorphic form "A", and the crystals obtained are then isolated; wherein pseudopolymorphic form "A" has an X-ray diffraction pattern with at least two characteristic peaks at 8.5 +/- 0.5 degrees 2θ and at 15.3 +/- 0.5 degrees 2θ.

5. Method according to claim 4, **characterized in that** it is carried out in the presence of seed crystals of the polymorphic form "B" and a final proportion of water less than 2%, by introducing the solution of sodium 2-ethylhexanoate over a period of 1 to 7 hours and operating at a temperature of 30 to 35°C.

6. Method for the preparation of the sodium salt according to claim 1, **characterized in that** a solution of the tetrabutylammonium salt of (1R,2S,5R)-7-oxo-6-sulfooxy-1,6-diazabicyclo[3.2.1]octane-2-carboxamide in pure ethanol is added to an ethanol/water mixture of sodium 2-ethylhexanoate in such a way that the final proportion of water is from 0 to 5 wt.% of the solvent, operating at a temperature of from 10 to 40°C, in the presence of seed crystals of the polymorphic form "B" or of the pseudopolymorphic form "A", and the crystals obtained are then isolated; wherein pseudopolymorphic form "A" has an X-ray diffraction pattern with at least two characteristic peaks at 8.5 +/- 0.5 degrees 2θ and at 15.3 +/- 0.5 degrees 2θ.

7. Method according to claim 6, **characterized in that** it is carried out in the presence of seed crystals of the polymorphic form "B" and a final proportion of water less than 2%, by introducing the solution of the tetrabutylammonium salt of (1R,2S,5R)-7-oxo-6-sulfooxy-1,6-diazabicyclo[3.2.1]octane-2-carboxamide over a period of 1 to 7 hours and operating at a temperature of 30 to 35°C.

8. The polymorphic form "B" of the sodium salt of (1R,2S,5R)-7-oxo-6-sulfooxy-1,6-diazabicyclo[3.2.1]octane-2-carboxamide, as claimed in claim 1, in combination with ceftazidime for use in the treatment of bacterial infections.

## Patentansprüche

1. Natriumsalz von (1R,2S,5R)-7-Oxo-6-sulfooxy-1,6-diazabicyclo[3.2.1]octan-2-carboxamid der Formel (I) 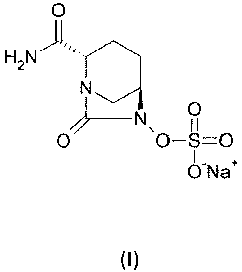 in kristalliner Form, wasserfrei, **dadurch gekennzeichnet, dass** das Salz in seiner polymorphen Form "B" vorliegt, und dass es ein Röntgenbeugungsmuster mit wenigstens fünf charakteristischen Peaks bei 13,0 +/- 0,5 Grad 2θ, bei 16,5 +/- 0,5 Grad 2θ, bei 17,2 +/- 0,5 Grad 2θ, bei 17,5 +/-0,5 Grad 2θ und bei 22,3 +/- 0,5 Grad 2θ mit zwei spezifischen Peaks bei 10,4 +/- 0,5 Grad 2θ und bei 13,0 +/-0,5 Grad 2θ hat.

2. Pharmazeutische Zusammensetzung, **dadurch gekennzeichnet, dass** sie als Inhaltsstoff das Natriumsalz in kristalliner Form gemäß Anspruch 1 und gegebenenfalls ein pharmazeutisch annehmbares Exzipiens enthält.

3. Pharmazeutische Zusammensetzung gemäß Anspruch 2, **dadurch gekennzeichnet, dass** sie des Weiteren als Inhaltsstoff ein antibakterielles Medikament des Beta-Lactamin-Typs enthält.

4. Verfahren zur Herstellung des Natriumsalzes gemäß Anspruch 1, **dadurch gekennzeichnet, dass** eine Lösung von Natrium-2-ethylhexanoat in reinem Ethanol zu einer Lösung des Tetrabutylammoniumsalzes von (1R,2S,5R)-7-Oxo-6-sulfooxy-1,6-diazabicyclo[3.2.1]octan-2-carboxamid, in einem Ethanol/Wassergemisch, auf so eine Weise, dass der endgültige Anteil an Wasser von 0 bis 5 Gew.-% des Lösungsmittels reicht, gegeben wird, wobei bei einer Temperatur von 10 bis 40°C gearbeitet wird, in der Gegenwart von Impfkristallen der polymorphen Form "B" oder der pseudopolymorphen Form "A", und die erhaltenen Kristalle dann isoliert werden; wobei die pseudopolymorphe Form "A" ein Röntgenbeugungsmuster mit wenigstens zwei charakteristischen Peaks bei 8,5 +/-0,5 Grad 2θ und bei 15,3 +/- 0,5 Grad 2θ besitzt.

5. Verfahren gemäß Anspruch 4, **dadurch gekennzeichnet, dass** es in der Gegenwart von Impfkristallen der polymorphen Form "B" und einem endgültigen Anteil von Wasser von weniger als 2% durchgeführt wird, indem die Lösung von Natrium-2-ethylhexanoat über einen Zeitraum von 1 bis 7 Stunden eingeführt wird und bei einer Temperatur von 30 bis 35°C gearbeitet wird.

6. Verfahren zur Herstellung des Natriumsalzes gemäß Anspruch 1, **dadurch gekennzeichnet, dass** eine Lösung des Tetrabutylammoniumsalzes von (1R,2S,5R)-7-Oxo-6-sulfooxy-1,6-diazabicyclo[3.2.1]octan-2-carboxamid in reinem Ethanol zu einem Ethanol/Wassergemisch von Natrium-2-ethylhexanoat auf so eine Weise, dass der endgültige Anteil von Wasser von 0 bis 5 Gew.-% des Lösungsmittels reicht, gegeben wird, wobei bei einer Temperatur von 10 bis 40°C gearbeitet wird, in der Gegenwart von Impfkristallen der polymorphen Form "B" oder der pseudopolymorphen Form "A", und die erhaltenen Kristalle dann isoliert werden; wobei die pseudopolymorphe Form "A" ein Röntgenbeugungsmuster mit wenigstens zwei charakteristischen Peaks bei 8,5 +/- 0,5 Grad 2θ und bei 15,3 +/- 0,5 Grad 2θ besitzt.

7. Verfahren gemäß Anspruch 6, **dadurch gekennzeichnet, dass** es in der Gegenwart von Impfkristallen der polymorphen Form "B" und einem endgültigen Anteil von Wasser von weniger als 2% durchgeführt wird, indem die Lösung des Tetrabutylammoniumsalzes von (1R,2S,5R)-7-Oxo-6-sulfooxy-1,6-diazabicyclo[3.2.1]octan-2-carboxamid über einen Zeitraum von 1 bis 7 Stunden eingeführt wird und bei einer Temperatur von 30 bis 35°C gearbeitet wird.

8. Polymorphe Form "B" des Natriumsalzes von (1R,2S,5R)-7-Oxo-6-sulfooxy-1,6-diazabicyclo[3.2.1]octan-2-carboxamid, wie in Anspruch 1 beansprucht, in Kombination mit Ceftazidim zur Verwendung in der Behandlung von bakteriellen Infektionen.

## Revendications

1. Sel de sodium du (1R,2S,5R)-7-oxo-6-sulfooxy-1,6-diazabicyclo[3.2.1]octane-2-carboxamide de formule (I) 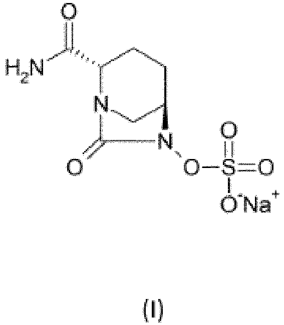 sous forme cristallisée, anhydre, **caractérisé en ce que** le sel est sous sa forme polymorphe « B », présentant un diagramme de diffraction des rayons X avec au moins cinq pics caractéristiques à 13,0 +/- 0,5 degrés 2θ, à 16,5 +/- 0,5 degrés 2θ, à 17,2 +/- 0,5 degrés 2θ, à 17,5 +/- 0,5 degrés 2θ, à 22,3 +/- 0,5 degrés 2θ et deux pics spécifiques à 10,4 +/- 0,5 degrés 2θ et à 13,0 +/- 0,5 degrés 2θ.

2. Composition pharmaceutique, **caractérisée en ce qu'**elle contient, comme ingrédient, le sel de sodium sous forme cristallisée selon la revendication 1 et, le cas échéant, un excipient pharmaceutiquement acceptable.

3. Composition pharmaceutique selon la revendication 2, **caractérisée en ce qu'**elle contient en outre, comme ingrédient, un médicament antibactérien du type bêta-lactamine.

4. Procédé de préparation du sel de sodium selon la revendication 1, **caractérisé en ce qu'**une solution de 2-éthylhexanoate de sodium dans l'éthanol pur est ajoutée à une solution du sel de tétrabutylammonium du (1R,2S,5R)-7-oxo-6-sulfooxy-1,6-diazabicyclo[3.2.1]-octane-2-carboxamide, dans un mélange éthanol/eau de manière à ce que la proportion finale d'eau soit de 0 à 5 % en poids du solvant, en opérant à une température de 10 à 40 °C, en présence de germes cristallins de la forme polymorphe « B » ou de la forme pseudopolymorphe « A », et les cristaux obtenus sont ensuite isolés ; dans lequel la forme pseudopolymorphe « A » présente un diagramme de diffraction des rayons X avec au moins deux pics caractéristiques à 8,5 +/- 0,5 degrés 2θ et à 15,3 +/- 0,5 degrés 2θ.

5. Procédé selon la revendication 4, **caractérisé en ce qu'**il est mis en oeuvre en présence de germes cristallins de la forme polymorphe « B » et d'une proportion finale d'eau inférieure à 2 %, en introduisant la solution de 2-éthylhexanoate de sodium sur une période de 1 à 7 heures et en opérant à une température de 30 à 35 °C.

6. Procédé de préparation du sel de sodium selon la revendication 1, **caractérisé en ce qu'**une solution du sel de tétrabutylammonium du (1R,2S,5R)-7-oxo-6-sulfooxy-1,6-diazabicyclo[3.2.1]octane-2-carboxamide dans l'éthanol pur est ajoutée à un mélange éthanol/eau de 2-éthylhexanoate de sodium de manière à ce que la proportion finale d'eau soit de 0 à 5 % en poids du solvant, en opérant à une température de 10 à 40 °C, en présence de germes cristallins de la forme polymorphe « B » ou de la forme pseudopolymorphe « A », et les cristaux obtenus sont ensuite isolés ; dans lequel la forme pseudopolymorphe « A » présente un diagramme de diffraction des rayons X avec au moins deux pics caractéristiques à 8,5 +/- 0,5 degrés 2θ et à 15,3 +/- 0,5 degrés 2θ.

7. Procédé selon la revendication 6, **caractérisé en ce qu'**il est mis en oeuvre en présence de germes cristallins de la forme polymorphe « B » et d'une proportion finale d'eau inférieure à 2 %, en introduisant la solution du sel de tétrabutylammonium du (1R,2S,5R]-7-oxo-6-sulfooxy-1,6-diazabicyclo[3.2.1]-octane-2-carboxamide sur une période de 1 à 7 heures et en opérant à une température de 30 à 35 °C.

8. Forme polymorphe « B » du sel de sodium du (1R,2S,5R)-7-oxo-6-sulfooxy-1,6-diazabicyclo[3.2.1]-octane-2-carboxamide, tel que revendiqué dans la revendication 1, en combinaison avec de la ceftazidime à des fins d'utilisation dans le traitement des infections bactériennes.
